(19) Europäisches Patentamt
European Patent Office
Office européen des brevets

(11) Veröffentlichungsnummer: **0 371 467 A2**

(12) **EUROPÄISCHE PATENTANMELDUNG**

(21) Anmeldenummer: **89121966.9**

(22) Anmeldetag: **28.11.89**

(51) Int. Cl.5: **C07F 5/02, C07K 5/00, A61K 37/02, A61K 31/69**

Patentansprüche für folgende Vertragsstaaten: ES + GR.

(30) Priorität: **01.12.88 DE 3840452**

(43) Veröffentlichungstag der Anmeldung: **06.06.90 Patentblatt 90/23**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Kleemann, Heinz-Werner, Dr.**
**Jahnstrasse 6**
**D-6092 Kelsterbach(DE)**
Erfinder: **Urbach, Hansjörg, Dr.**
**Le Lavandoustrasse 41**
**D-6242 Kronberg/Taunus(DE)**
Erfinder: **Ruppert, Dieter, Dr.**
**Schreyerstrasse 30**
**D-6242 Kronberg/Taunus(DE)**
Erfinder: **Schölkens, Bernward, Dr.**
**Hölderlinstrasse 62**
**D-6233 Kelkheim/Taunus(DE)**

(54) **Beta-Amino-Boronsäure-Derivate.**

(57) Die Erfindung betrifft Verbindungen der Formel I

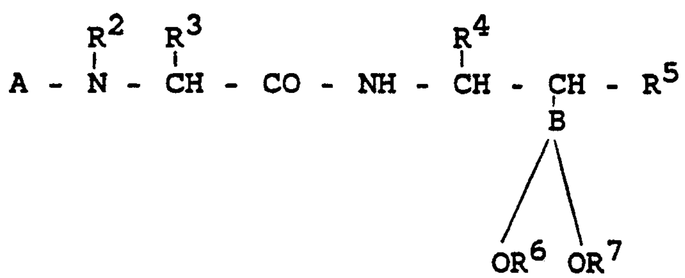

. (I)

in welcher
A einen Rest der Formeln II, III oder IV bedeutet

EP 0 371 467 A2

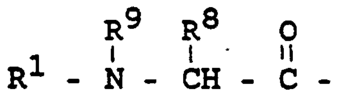

(II)

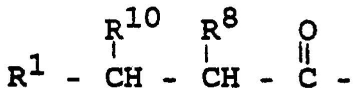

(III)

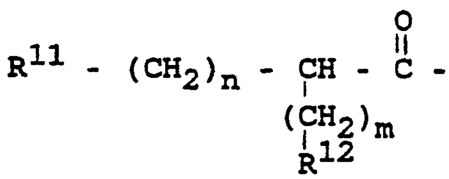

(IV)

und $R^1$ bis $R^{12}$ wie in der Beschreibung angegeben definiert sind,
sowie Verfahren zu ihrer Herstellung, diese enthaltende Mittel und deren Verwendung.

## β-Amino-Boronsäure-Derivate

Die Erfindung betrifft Verbindungen der Formel I

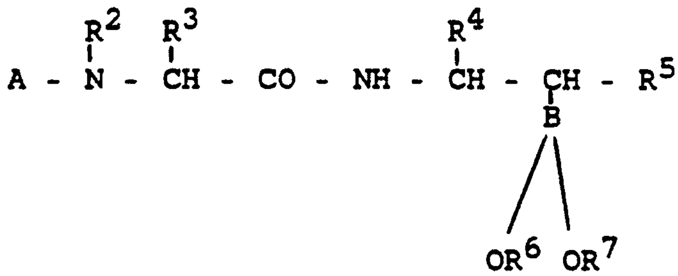

(I)

in welcher
A einen Rest der Formeln II, III oder IV bedeutet

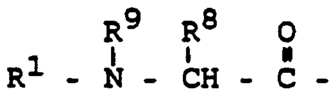

(II)

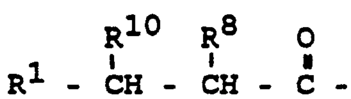

(III)

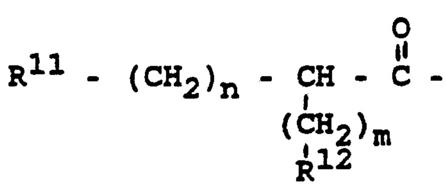

(IV)

worin
$R^1$ $a_1$) Wasserstoff, ($C_1$-$C_{12}$)-Alkyl, das gegebenenfalls ein-oder zweifach ungesättigt ist und das gegebenenfalls durch bis zu 3 gleiche oder verschiedene Reste aus der Reihe Hydroxy, ($C_1$-$C_7$)-Alkoxy, Carbamoyl, ($C_1$-$C_8$)-Alkanoyloxy, Carboxy, ($C_1$-$C_7$)-Alkoxycarbonyl, F, Cl, Br, I, Amino, Amidino, das gegebenenfalls durch einen, zwei oder drei ($C_1$-$C_8$)-Alkylreste substituiert sein kann, Guanidino, das gegebenenfalls durch einen, zwei, drei oder vier ($C_1$-$C_8$)-Alkylreste substituiert sein kann, ($C_1$-$C_7$)-Alkylamino, Di-($C_1$-$C_7$)-alkylamino, ($C_1$-$C_5$) -Alkoxycarbonylamino, ($C_7$-$C_{15}$)-Aralkoxycarbonylamino und 9-Fluorenylmethoxycarbonylamino substituiert ist;
mono-, bi- oder tricyclisches ($C_3$-$C_{18}$)-Cycloalkyl, ($C_3$-$C_{18}$)-Cycloalkyl-($C_1$-$C_6$)-alkyl oder ($C_6$-$C_{14}$)-Aryl, wobei der Cycloalkylteil gegebenenfalls durch ($C_1$-$C_6$)-Alkyl substituiert ist und Aryl gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino und Trifluormethyl substituiert ist;
($C_6$-$C_{14}$)-Aryl-($C_1$-$C_6$)-alkyl, worin der Arylteil gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino, ($C_1$-$C_7$)-Alkylamino, Di-($C_1$-$C_7$)-alkylamino, Carboxy, Carboxymethoxy, Amino-($C_1$-$C_7$)-alkyl, ($C_1$-$C_7$)-Alkylamino-($C_1$-$C_7$)-alkyl, Di-($C_1$-$C_7$)-alkylamino-($C_1$-$C_7$)-alkyl, ($C_1$-$C_7$)-Alkoxycarbonylmethoxy, Carbamoyl, Sulfamoyl, ($C_1$-$C_7$)-Alkoxysulfonyl, Sulfo- und Guanidino-($C_1$-$C_8$)-alkyl substituiert ist; oder für den Rest eines 5- oder 6-gliedrigen monocyclischen oder 9- oder 10-gliedrigen bicyclischen teilweise oder vollständig hydrierten Heteroaromaten, mit mindestens 1 C-Atom, 1 - 4 N-Atomen und/oder 1 - 2 S-Atomen

und/oder 1 - 2 O-Atomen als Ringglieder steht, und der gegebenenfalls wie ($C_6$-$C_{14}$)-Aryl unter $a_1$) definiert, mono-, di- oder trisubstituiert ist, bedeutet oder

$a_2$) einen Rest der Formel V bedeutet

$R^1$ - W , (V)

worin $R^1$ wie $R^1$ unter $a_1$) definiert ist und W für

-CO-, -O-CO-, -$SO_2$-, SO-, -NH-$SO_2$-, -NH-CO-, -CH(OH)-oder -N(OH)- steht;

$R^2$ Wasserstoff oder ($C_1$-$C_8$)-Alkyl bedeutet, oder zusammen mit $R^3$ und den diese tragenden Atomen ein mono- oder bicyclisches, gesättigtes oder teilweise ungesättigtes Ringsystem mit 5-12 Ringgliedern bildet;

$R^3$ und $R^8$ unabhängig voneinander wie $R^1$ und $a_1$) definiert sind oder zusammen mit $R^2$ bzw. mit $R^9$ und den diese tragenden Atomen Ringsysteme mit 5-12 Ringgliedern bilden wie oben definiert;

$R^4$ ($C_3$-$C_{12}$)-Alkyl, mono-, bi- oder tricyclisches ($C_3$-$C_{18}$)-Cycloalkyl, ($C_3$-$C_{18}$)-Cycloalkylmethyl, ($C_3$-$C_{18}$)-Cycloalkylethyl, wobei der Cycloalkylteil gegebenenfalls durch ($C_1$-$C_6$)-Alkyl substiuiert ist; Dithiolanyl; ($C_6$-$C_{14}$)-Arylmethyl; Dithiolanylmethyl; Dithiolanylethyl; Dithianyl; Dithianylmethyl oder Dithianylethyl bedeutet;

$R^5$ Wasserstoff; Azido; Azido-($C_1$-$C_4$)-alkyl; ($C_1$-$C_{12}$)-Alkyl, das gegebenenfalls durch Hydroxy, Azido oder Hydroxy und Azido substituiert sein kann; ($C_3$-$C_{12}$)-Cycloalkyl; ($C_3$-$C_{12}$)-Cycloalkyl-($C_1$-$C_6$)-alkyl; $C_3$-$C_{12}$)-Cycloalkylsulfonyl-($C_1$-$C_6$)-alkyl; ($C_1$-$C_6$)-Alkylsulfonyl-($C_1$-$C_6$)-alkyl; ($C_6$-$C_{14}$)-Aryl, ($C_6$-$C_{14}$)-Aryl-($C_1$-$C_6$)-alkyl, wobei Aryl auch für den Rest eines 5- oder 6-gliedrigen monocyclischen oder 9-oder 10-gliedrigen bicyclischen Heteraromaten mit mindestens 1 C-Atom, 1-4 N-Atomen und/oder 1-2 S-Atomen und/oder 1-2 O-Atomen als Ringglieder stehen kann, der auch teilweise oder vollständig hydriert sein kann, ebenfalls ist es möglich, daß Aryl und Heteroaryl wie unter $a_1$) definiert substituiert sein kann,

$R^6$ und $R^7$ unabhängig voneinander Wasserstoff oder ($C_1$-$C_6$)-Alkyl bedeuten; oder zusammen mit dem Boratom und den Sauerstoffatomen ein mono-, bi- oder tricyclisches, gesättigtes oder teilweise ungesättigtes, mono-, di-, tri- oder tetra-($C_1$-$C_6$)-alkyliertes oder -phenyliertes Ringsystem mit 5-18 Ringgliedern, das außer dem Boratom, den Sauerstoffatomen und dem/den Kohlenstoffatom(en) noch ein -O- Glied, ein -$NR^{13}$-Glied oder ein -$CR^{14}R^{15}$-Glied enthalten kann, bilden;

$R^9$ für Wasserstoff oder ($C_1$-$C_8$)-Alkyl steht, oder zusammen mit $R^8$ und den diese tragenden Atomen ein mono- oder bicyclisches, gesättigtes oder teilweise ungesättigtes Ringsystem mit 5-12 Ringgliedern bildet;

$R^{10}$ Wasserstoff oder ($C_1$-$C_8$)-Alkyl ist, oder zusammen mit $R^1$ oder $R^8$ und den diese tragenden Atomen ein mono- oder bicyclisches, gesättigtes oder teilweise ungesättigtes Ringsystem mit 5-12 Ringgliedern bildet, das außer Kohlenstoff noch 1 Schwefelatom enthalten kann, welches gegebenenfalls zum Sulfoxid oder Sulfon oxidiert sein kann;

$R^{11}$ und $R^{12}$ unabhängig voneinander Wasserstoff, Hydroxy oder ($C_6$-$C_{14}$)-Aryl bedeuten, wobei Aryl gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino, ($C_1$-$C_7$)-Alkylamino, Di-($C_1$-$C_7$)-alkylamino, Carboxy, Carboxymethoxy, Amino-($C_1$-$C_7$)-alkyl, ($C_1$-$C_7$)-Alkylamino-($C_1$-$C_7$)-alkyl, Di- ($C_1$-$C_7$)-alkylamino-($C_1$-$C_7$)-alkyl, ($C_1$-$C_7$)-Alkoxycarbonylmethoxy, Carbamoyl, Sulfamoyl, ($C_1$-$C_7$)-Alkoxysulfonyl, Sulfo- und Guanidino-($C_1$-$C_8$)-alkyl substituiert ist; oder für den Rest eines 5- oder 6-gliedrigen monocyclischen oder 9- oder 10-gliedrigen bicyclischen, gegebenenfalls teilweise oder vollständig hydrierten Heteroaromaten, mit mindestens 1 C-Atom, 1 - 4 N-Atomen und/oder 1 - 2 S-Atomen und/oder 1 - 2 O-Atomen als Ringglieder stehen, der gegebenenfalls wie oben ($C_6$-$C_{14}$)-Aryl mono- oder disubstituiert ist,

n und m unabhängig voneinander 0, 1, 2, 3 und 4 sein können,

$R^{13}$ Wasserstoff oder ($C_1$-$C_{12}$)-Alkyl, das gegebenenfalls ein- bis zweifach ungesättigt ist und das gegebenenfalls durch bis zu 3 gleiche oder verschiedene Reste aus der Reihe Hydroxy, ($C_1$-$C_7$)-Alkoxy, Amino, Mono- oder Di-($C_1$-$C_7$)-alkylamino substituiert ist, bedeutet,

$R^{14}$ und $R^{15}$ unabhängig voneinander Wasserstoff, ($C_1$-$C_8$)-Alkyl, Hydroxymethyl, 2-Hydroxyethyl, (3-Hydroxysulfonyl, 2-hydroxypropyl)amino, (2-Hydroxysulfonylethyl)amino, (2-Hydroxysulfonylpropyl)amino, (Carboxymethyl)amino, Bis(2-hydroxyethyl)amino bedeuten

sowie deren physiologisch verträgliche Salze.

Unter einem Rest eines 5- oder 6-gliedrigen monocyclischen oder 9- oder 10-gliedrigen bicyclischen Heteroaromaten mit mindestens 1 C-Atom, 1 - 4 N-Atomen und/oder 1 - 2 S-Atomen und/oder 1 - 2 O-Atomen als Ringglieder werden Reste von Heteroaromaten verstanden, wie sie beispielsweise in Katritzky, Lagowski, Chemie der Heterocyclen, Berlin, Heidelberg 1968 definiert sind. Der Heteroaromaten-Rest kann durch einen, zwei oder drei, vorzugsweise einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino oder Trifluormethyl substituiert sein. Monocyclische Heteroaromaten sind z.B. Thiophen, Furan, Pyrrol, Imidazol, Pyrazol, Pyridin, Pyrazin, Pyrimidin, Pyridazin, 1,2,4-Triazol, Thiazol, Tetrazol, Isothiazol, Oxazol und Isoxazol. Bicyclische Heteroaromaten sind z.B. Benzothiophen, Benzofuran, Indol, Isoindol, Indazol, Benzimidazol,

Chinolin, Isochinolin, Phthalazin, Chinoxalin, Chinazolin und Cinnolin. Entsprechendes gilt für von Heteroaryl abgeleitete Reste, wie z.B. ganz oder teilweise hydriertes Heteroaryl, u.a. auch z.B. Benzodioxolan, Heteroaryloxy, Heteroarylthio und Heteroaryl-alkyl.

Alkyl kann geradkettig oder verzweigt sein. Entsprechendes gilt für davon abgeleitete Reste, wie z.B. Alkoxy, Alkylthio, Alkylamino, Dialkylamino, Alkylsulfinyl, Alkylsulfonyl, Alkanoyl und Aralkyl.

($C_6$-$C_{14}$)-Aryl ist beispielsweise Phenyl, Naphthyl, Biphenylyl oder Fluorenyl; bevorzugt sind Phenyl und Naphthyl. Entsprechendes gilt für davon abgeleitete Reste, wie z.B. Aryloxy, Aroyl, Aralkyl und Aralkyloxy. Unter Aralkyl versteht man einen mit ($C_1$-$C_6$)-Alkyl verknüpften unsubstituierten oder substituierten ($C_6$-$C_{14}$)-Aryl-Rest, wie z.B. Benzyl, 1- und 2-Naphthylmethyl, Halobenzyl und Alkoxybenzyl, wobei Aralkyl jedoch nicht auf die genannten Reste beschränkt wäre.

Unter Salzen von Verbindungen der Formel I sind insbesondere pharmazeutisch verwendbare oder nichttoxische Salze zu verstehen.

Solche Salze werden beispielsweise von Verbindungen der Formel I, welche saure Gruppen, z.B. Carboxy, enthalten, mit Alkali- oder Erdalkalimetallen gebildet, wie Na, K, Mg und Ca, sowie mit physiologisch verträglichen organischen Aminen, wie z.B. Triethylamin und Tri-(2-hydroxy-ethyl)amin.

Verbindungen der Formel I, welche basische Gruppen, z.B. eine Aminogruppe oder eine Guanidinogruppe, enthalten, bilden Salze mit anorganischen Säuren, wie z.B. Salzsäure, Schwefelsäure oder Phosphorsäure und mit organischen Carbon- oder Sulfonsäuren, wie z.B. Essigsäure, Citronensäure, Benzoesäure, Maleinsäure, Fumarsäure, Weinsäure und p-Toluolsulfonsäure.

Bevorzugt sind Verbindungen der Formel I, in denen die Reste wie folgt definiert sind:

A ist wie auf der Seite 1 definiert,

$R^1$ bedeutet vorzugsweise Wasserstoff oder steht für ($C_1$-$C_{10}$)-Alkyl; Cyclopentyl; Cyclohexyl; Cyclopentyl-($C_1$-$C_{10}$)-alkyl; Cyclohexyl-($C_1$-$C_{10}$)-alkyl; gegebenenfalls substituiertes Phenyl-($C_1$-$C_8$)-alkyl; 2-Pyridyl-($C_1$-$C_8$)-alkyl; 3-Pyridyl-($C_1$-$C_8$)-alkyl; 4-Pyridyl-($C_1$-$C_8$)-alkyl; $H_2N$-($C_1$-$C_{10}$)-Alkyl; HO-($C_1$-$C_{10}$)-Alkyl; ($C_1$-$C_4$)-Alkoxy-($C_1$-$C_{10}$)-alkyl; ($C_1$-$C_4$)-Alkoxycarbonyl-($C_1$-$C_{10}$)-alkyl; ($C_1$-$C_8$)-Alkylsulfonyl; ($C_1$-$C_8$)-Alkylsulfinyl; ( $C_1$-$C_8$) -Hydroxyalkylsulfonyl; ($C_1$-$C_8$)-Hydroxy-alkylsulfinyl; Hydroxy-($C_1$-$C_{10}$)-alkanoyl, wie 2-Hydroxypropionyl, 3-Hydroxypropionyl, 3-Hydroxybutyryl oder 2-Hydroxy-3-methyl-butyryl; ($C_1$-$C_8$)-Alkanoyloxy-($C_1$-$C_{10}$)-alkyl; ($C_1$-$C_{11}$)-Alkanoyl, wie n-Decanoyl, Formyl, Acetyl, Propionyl, Pivaloyl, Isovaleryl oder Isobutyryl; gegebenenfalls geschütztes Amino-($C_1$-$C_{11}$)-alkanoyl, wie (3-Amino,3,3 -dimethyl)propionyl, 4-Aminobutyryl, 5-Aminopentanoyl, 6-Aminohexanoyl, 4-N-tert.-Butoxycarbonylaminobutyryl, 5-N-tert.-Butoxycarbonylaminopentanoyl oder 6-N-tert.-Butoxycarbonylaminohexanoyl;

Di-($C_1$-$C_7$)-alkylamino-($C_2$-$C_{11}$)-alkanoyl, wie Dimethylaminoacetyl; Piperidino-4-carbonyl;

Morpholino-4-carbonyl; ($C_3$-$C_9$)-Cycloalkylcarbonyl, wie Cyclopropylcarbonyl, Cyclobutylcarbonyl, Cyclopentylcarbonyl oder Cyclohexylcarbonyl;

($C_6$-$C_{10}$)-Aryl-($C_2$-$C_{11}$)-alkanoyl, wie Phenylacetyl, Phenylpropanoyl oder Phenylbutanoyl; 2-Pyridyl-($C_1$-$C_8$)-alkanoyl; 3-Pyridyl-($C_1$-$C_8$)-alkanoyl;

4-Pyridyl-($C_1$-$C_8$)-alkanoyl; gegebenenfalls durch Halogen, ($C_1$-$C_7$)-Alkyl, ($C_1$-$C_7$)-Alkoxy oder ($C_1$-$C_7$)-Alkoxycarbonyl substituiertes Benzoyl, wie 4-Chlorbenzoyl, 4-Methylbenzoyl, 2-Methoxycarbonylbenzoyl oder 4-Methoxybenzoyl; Pyrrolyl-2-carbonyl, Pyridyl-3-carbonyl; Benzolsulfonyl;

($C_1$-$C_{10}$)-Alkoxycarbonyl, wie Methoxycarbonyl, Ethoxycarbonyl, Isobutoxycarbonyl oder tert.-Butoxycarbonyl; substituiertes ($C_1$-$C_{10}$)-Alkoxycarbonyl, wie 2-(Trimethylsilyl) ethoxycarbonyl, 2,2,2-Trichlorethoxycarbonyl oder 1,1-Dimethyl-2,2,2-trichlorethoxycarbonyl;

($C_6$-$C_{14}$)-Aryl-($C_1$-$C_6$)-alkoxycarbonyl, wie Benzyloxycarbonyl, 1- oder 2-Naphthylmethoxycarbonyl oder 9-Fluorenylmethoxycarbonyl; oder $R^1$ bildet bevorzugt gemeinsam mit $R^{10}$ ein mono- oder bicyclisches, gesättigtes oder teilweise ungesättigtes Ringsystem mit 5-12 Ringgliedern, das außer Kohlenstoff noch 1 Schwefelatom enthalten kann, welches gegebenenfalls zum Sulfoxid oder Sulfon oxydiert sein kann,

$R^2$ ist bevorzugt Wasserstoff, Methyl oder Ethyl oder bildet zusammen mit $R^3$ und den diese tragenden Atomen bevorzugt Pyrrolidin oder Piperidin, die jeweils noch mit Cyclopentyl, Cyclohexyl oder Phenyl anneliert sein können,

$R^3$ und $R^8$ sind unabhängig voneinander bevorzugt Wasserstoff; ($C_1$-$C_{10}$)-Alkyl, daß gegebenenfalls ein- oder zweifach ungesättigt ist und das gegebenenfalls durch bis zu 3 gleiche oder verschiedene Reste aus der Reihe Hydroxy, ($C_1$-$C_7$)-Alkoxy, ($C_1$-$C_7$)-Alkanoyloxy, Carboxy, ($C_1$-$C_7$)-Alkoxycarbonyl, Cl, Br, Amino, Amidino, Guanidino, Carbamoyl, ($C_1$-$C_5$)-Alkoxycarbonylamino, ($C_6$-$C_{15}$)-Aralkoxycarbonylamino und 9-Fluorenylmethoxycarbonylamino substituiert ist; ($C_3$-$C_{12}$)-Cycloalkyl, ($C_3$-$C_{12}$)-Cycloalkyl-($C_1$-$C_3$)-alkyl, mono- oder bicyclisches ($C_6$-$C_{14}$)-Aryl-($C_1$-$C_3$)-Alkyl, das gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino und Trifluormethyl substituiert ist; oder stehen bevorzugt für ($C_1$-$C_3$)-Alkyl, substituiert mit dem Rest eines 5- oder 6-gliedrigen monocyclischen oder 9- oder 10-gliedrigen bicyclischen, gegebenenfalls

teilweise oder vollständig hydrierten Heteroatomen, mit mindestens 1 C-Atom, 1 - 4 N-Atomen und/oder 1 - 2 S-Atomen und/oder 1 - 2 O-Atomen als Ringglieder, der gegebenenfalls wie auf Seite 2 für den Arylteil beschrieben mono- oder disubstituiert ist, oder bilden zusammen mit $R^2$ bzw. $R^9$ Ringsysteme, wie vorstehend unter $R^2$ beschrieben;

$R^4$ ist bevorzugt ($C_3$-$C_{12}$)-Alkyl; mono- bi- oder tricyclisches ($C_3$-$C_{18}$)-Cycloalkyl oder ($C_3$-$C_{18}$)-Cycloalkylmethyl, wobei der Cycloalkylteil gegebenenfalls durch ($C_1$-$C_4$)-Alkyl substituiert ist; ($C_6$-$C_{14}$)-Arylmethyl; Dithiolanyl; Dithiolanylmethyl; Dithianyl und Dithianylmethyl;

$R^5$ steht bevorzugt für Wasserstoff, Azido, Azido-($C_1$-$C_4$)-alkyl, ($C_1$-$C_{12}$)-Alkyl, ($C_3$-$C_{12}$)-Cycloalkyl, ($C_3$-$C_{12}$)-Cycloalkyl-($C_1$-$C_6$)-alkyl, (2-Pyridyl)-($C_1$-$C_4$)-alkyl, (3-Pyridyl)-($C_1$-$C_4$)-alkyl, (4-Pyridyl)-($C_1$-$C_4$)-alkyl, Imidazol-2-yl, Imidazol-1-yl, Imidazol-4-yl, (Imidazol-2-yl)-($C_1$-$C_4$)-alkyl, (Imidazol-1-yl)-($C_1$-$C_4$)-alkyl, (Imidazol-4-yl)-($C_1$-$C_4$)-alkyl, [1-($C_1$-$C_6$)-Alkylimidazol-2-yl]-($C_1$-$C_4$)-alkyl, (Imidazolin-2-yl)-($C_1$-$C_4$)-alkyl, [1-($C_1$-$C_6$)-Alkylimidazolin-2-yl]-($C_1$-$C_4$)-alkyl;

$R^6$ und $R^7$ sind unabhängig voneinander bevorzugt Wasserstoff oder bilden zusammen mit dem Boratom und den Sauerstoffatomen ein mono-, bi- oder tricyclisches, gesättigtes oder teilweise ungesättigtes mono-, di-, tri-oder tetraalkyliertes oder -phenyliertes Ringsystem mit 5-18 Ringgliedern, das außer dem Boratom, den Sauerstoffatomen und dem (den) Kohlenstoffatom(en) noch ein -O-Glied, ein -$NR^{13}$-Glied oder ein -$CR^{14}R^{15}$-Glied enthalten kann;

$R^9$ ist bevorzugt Wasserstoff, Methyl oder Ethyl oder bildet zusammen mit $R^{°}$ und den diese tragenden Atomen Pyrrolidin oder Piperidin, die jeweils zusätzlich mit Cyclopentyl, Cyclohexyl oder Phenyl anneliert sein können;

$R^{10}$ ist bevorzugt Wasserstoff oder Methyl, oder bildet gemeinsam mit $R^1$ oder $R^8$ und den diese tragenden Atomen ein mono- oder bicyclisches gesättigtes oder teilweise ungesättigtes Ringsystem mit 5-12 Ringgliedern, das außer Kohlenstoff noch 1 Schwefelatom enthalten kann, welches gegebenenfalls zum Sulfoxid oder Sulfon oxidiert sein kann,

$R^{11}$ und $R^{12}$ sind unabhängig voneinander bevorzugt Wasserstoff, Hydroxy, Phenyl, 2- oder 3-Thienyl, 2-, 3- oder 4-Pyridyl, 1-, 2- oder 4-Imidazolyl, 1- oder 2-Naphthyl; 2- oder 3-Benzo[b]thienyl,

n und m können unabhängig voneinander 0, 1, 2, 3 und 4 bedeuten,

$R^{13}$ ist bevorzugt Wasserstoff oder ($C_1$-$C_{12}$)-Alkyl,

$R^{14}$ und $R^{15}$ bedeuten unabhängig voneinander bevorzugt Wasserstoff, Hydroxymethyl, 2-Hydroxyethyl, (3-Hydroxysulfonyl, 2-hydroxypropyl)amino, (2-Hydroxysulfonylethyl) amino, (2-Hydroxysulfonylpropyl)amino, (Carboxymethyl)amino, Bis(2-hydroxyethyl)amino.

$R^1$ bedeutet besonders bevorzugt ($C_1$-$C_8$)-Alkylsulfonyl;

($C_1$-$C_8$)-Alkylsulfinyl; ($C_1$-$C_8$)-Hydroxyalkylsulfonyl, insbesondere 2-Hydroxyethylsulfonyl oder 2-Hydroxypropylsulfonyl; Hydroxy-($C_1$-$C_{10}$)-alkanoyl, wie 2-Hydroxypropionyl, 3-Hydroxypropionyl, 3-Hydroxybutyryl oder 2-Hydroxy-3-methylbutyryl;

($C_1$-$C_8$)-Alkanoyloxy-($C_1$-$C_{10}$)-alkyl; ($C_1$-$C_{11}$)Alkanoyl, wie n-Decanoyl, Formyl, Acetyl, Propionyl, Pivaloyl, Isovaleryl oder Isobutyryl; Amino-($C_1$-$C_{11}$)-alkanoyl, wie (3-Amino, 3,3-dimethyl)propionyl), 4-Aminobutyryl, 5-Aminopentanoyl, 6-Aminohexanoyl; Di-($C_1$-$C_7$)-alkylamino-($C_2$-$C_{11}$)-alkanoyl, wie Dimethylaminoacetyl; Piperidino-4-carbonyl; Morpholino-4-carbonyl;

($C_3$-$C_9$)-Cycloalkylcarbonyl, wie Cyclopropylcarbonyl, Cyclobutylcarbonyl, Cyclopentylcarbonyl oder Cyclohexylcarbonyl; ($C_6$-$C_{10}$)-Aryl-($C_2$-$C_{11}$)-alkanoyl, wie Phenylacetyl, Phenylpropanoyl oder Phenylbutanoyl; 2-Pyridyl-($C_1$-$C_8$)-alkanoyl; 3-Pyridyl-($C_1$-$C_8$)-alkanoyl;

4-Pyridyl-($C_1$-$C_8$)-alkanoyl; gegebenenfalls durch Halogen, ($C_1$-$C_7$)-Alkyl, ($C_1$-$C_7$)-Alkoxy oder ($C_1$-$C_7$)-Alkoxycarbonyl substituiertes Benzoyl, wie 4-Chlorbenzoyl, 4-Methylbenzoyl, 2-Methoxycarbonylbenzoyl oder 4-Methoxybenzoyl; Pyrrolyl-2-carbonyl;

Pyridyl-3-carbonyl; Benzolsulfonyl;

($C_1$-$C_{10}$)-Alkoxycarbonyl, wie Methoxycarbonyl, Ethoxycarbonyl, Isobutoxycarbonyl oder tert.-Butoxycarbonyl; substituiertes ($C_1$-$C_{10}$)-Alkoxycarbonyl, wie 2-(Trimethylsilyl)-ethoxycarbonyl, 2,2,2-Trichlorethoxycarbonyl oder 1,1-Dimethyl-2,2,2-trichlorethoxycarbonyl;

($C_6$-$C_{14}$)-Aryl-($C_1$-$C_6$)-alkoxycarbonyl, wie Benzyloxycarbonyl, 1- oder 2-Naphthylmethoxycarbonyl oder 9-Fluorenylmethoxycarbonyl,

$R^2$ bedeutet besonders bevorzugt Wasserstoff oder Methyl oder bildet gemeinsam mit $R^3$ und der diese Reste tragenden -N-CH- Gruppe ein Tetrahydroisochinolin oder Azabicyclooctan-Gerüst,

$R^3$ und $R^8$ sind unabhängig voneinander besonders bevorzugt Wasserstoff, Methyl, Ethyl, Isopropyl, n-Propyl, n-Butyl, Isobutyl, sec.-Butyl, 3-Guanidinopropyl, Carbamoylmethyl, 2-Carbamoylethyl, Carboxymethyl, 2-Carboxyethyl, Mercaptomethyl, 2-(Methylthio)ethyl, (1-Mercapto,1-methyl)ethyl, Hydroxymethyl, 1-Hydroxyethyl, Amino, Aminomethyl, 2-Aminoethyl, 3-Aminopropyl, 4-Aminobutyl, N,N-Dimethylamino, Cyclohexylmethyl, Imidazol-4-yl-methyl, Benzyl, 2-Methylbenzyl, 3-Methylbenzyl, Indol-3-yl-methyl, 4-Hydroxybenzyl, 4-Methoxybenzyl, 3,4-Dihydroxybenzyl, 3,4-Dimethoxybenzyl, (Benzdioxolan-5-yl)methyl, 2-Thienyl, 2-Thienylmethyl, 2-(2-Thienyl)ethyl, 3-Thienyl, 3-Thienylmethyl, 2-(3-Thienyl)ethyl, 4-Chlorbenzyl, 2-(Methylsulfinyl)ethyl, 2-(Methylsulfonyl)ethyl, 2-Pyridylmethyl, 3-Pyridylmethyl, 4-Pyridylmethyl, Cyclohexyl, (1-Methyl-imidazol-4-yl)methyl, (3-Methyl-imidazol-4-yl)methyl, Phenyl, 1-Naphthylmethyl, 2-Naphthylmethyl, 2-Phenylethyl, 2-Thiazolylmethyl, 4-Thiazolylmethyl, 3-Pyrazolylmethyl, 4-Pyrimidinylmethyl, Indol-2-ylmethyl, 2-Benzo[b]thienylmethyl, 3-Benzo[b]thienylmethyl, 2-Furylmethyl,
oder bilden mit $R^2$ bzw. $R^9$ Ringsysteme wie vorstehend unter $R^2$ definiert,
$R^4$ ist besonders bevorzugt ($C_3$-$C_{12}$)-Alkyl; mono- oder bicyclisches ($C_3$-$C_{12}$)-Cycloalkyl oder ($C_3$-$C_{12}$)-Cycloalkylmethyl, wobei der Cycloalkylteil gegebenenfalls durch ($C_1$-$C_4$)-Alkyl substituiert ist; ($C_6$-$C_{10}$)-Arylmethyl; Dithiolanyl; Dithiolanylmethyl;
Dithianyl und Dithianylmethyl,
$R^5$ ist besonders bevorzugt Wasserstoff, Azido, Azidomethyl, 2-Azidoethyl, ($C_1$-$C_6$)-Alkyl, ($C_3$-$C_{12}$)-Cycloalkyl, ($C_3$-$C_{12}$)-Cycloalkyl-($C_1$-$C_3$)alkyl, 3-(2-Pyridyl)-propyl, 3-(3-Pyridyl)-propyl, 3-(4-Pyridyl)-propyl, Imidazol-2-yl, Imidazol-1-yl, Imidazol-4-yl, (Imidazol-2-yl)-propyl, (Imidazol-1-yl)-propyl, (Imidazol-4-yl)-propyl, [1-($C_1$-$C_4$)-Alkylimidazol-2-yl]-propyl, (Imidazolin-2-yl)-propyl,
$R^6$ und $R^7$ sind wie auf Seite 10 definiert,
$R^9$ bedeutet besonders bevorzugt Wasserstoff oder Methyl oder bildet zusammen mit $R^8$ und der diese Reste tragenden -N-CH- Gruppe ein Tetrahydroisochinolin- oder Azabicyclooctan-Gerüst,
$R^{10}$ bedeutet besonders bevorzugt Wasserstoff oder bildet zusammen mit $R^1$ und den diese tragenden Atomen ein mono-oder bicyclisches gesättigtes oder teilweise ungesättigtes Ringsystem mit 5-12 Ringgliedern, das außer Kohlenstoff noch 1 Schwefelatom enthält, welches besonders bevorzugt zum Sulfon oxydiert ist, oder bildet zusammen mit $R^8$ und den diese tragenden Atomen ein Thiochromansystem, dessen Schwefelatom besonders bevorzugt zum Sulfon oxydiert ist,
$R^{11}$ und $R^{12}$ sind unabhängig voneinander besonders bevorzugt Wasserstoff, Hydroxy, Phenyl, 2-Thienyl, 2-, 3- oder 4-Pyridyl, 1- oder 2-Imidazolyl, 1-Naphthyl, 2- oder 3-Benzo[b]thienyl,
n und m sind unabhängig voneinander besonders bevorzugt 0, 1 oder 2,
$R^{13}$ ist besonders bevorzugt Wasserstoff oder ($C_1$-$C_4$)-Alkyl,
$R^{14}$ und $R^{15}$ sind wie auf Seite 11 definiert,
Die Erfindung betrifft weiterhin ein Verfahren zur Herstellung von Verbindungen der Formel I, das dadurch gekennzeichnet ist, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

Fragmente einer Verbindung der Formel I mit einer endständigen Carboxylgruppe besitzen die nachstehenden Formeln VI bis VII

A - OH (VI)

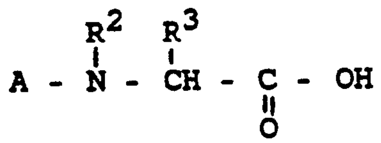

(VII)

Fragmente einer Verbindung der Formel I mit einer endständigen Aminogruppe besitzen die nachstehenden Formeln VIII bis X

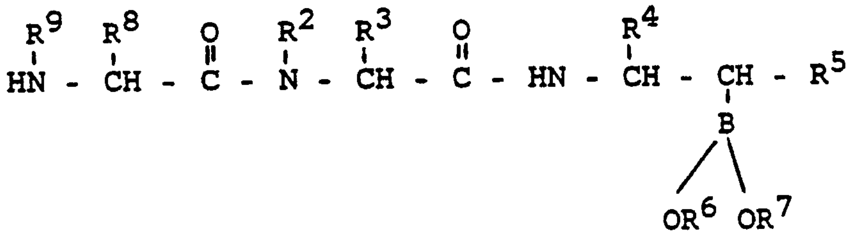

(VIII)

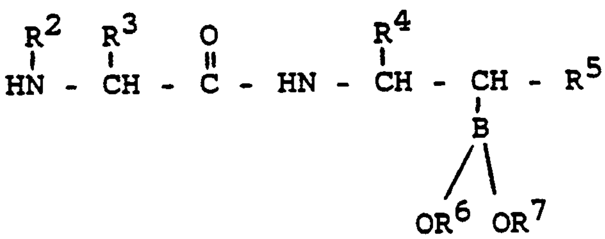

(IX)

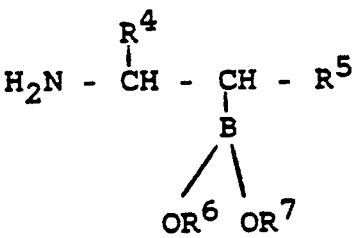

(X)

Methoden, die zur Herstellung einer Amidbindung geeignet sind, werden z.B. in Houben-Weyl, Methoden der organischen Chemie, Band 15/2; Bodanszky et al., Peptide Synthesis, 2nd ed. (Wiley & Sons, New York 1976) oder Gross, Meienhofer, The Peptides: Analysis, synthesis, biology (Academic Press, New York 1979) beschrieben. Vorzugsweise werden die folgenden Methoden herangezogen:
Aktivestermethode mit N-Hydroxy-succinimid, 1-Hydroxybenzotriazol oder 3-Hydroxy-4-oxo-3,4-dihydro-1,2,3-benzotriazin als Alkoholkomponente, Kupplung mit einem Carbodiimid wie Dicyclohexylcarbodiimid oder mit Propanphosphonsäureanhydrid und die Gemischt-Anhydrid-Methode mit Pivaloylchlorid oder Chlorameisensäureethylester oder -isobutylester.

Fragmente der Formel VI, sofern sie unter

a) Formel II fallen, werden nach den allgemein bekannten Methoden zur Herstellung von Aminosäuren synthetisiert;

b) Formel III fallen, werden ausgehend von den entsprechenden Aminosäuren synthetisiert, wobei deren Chiralitätszentrum erhalten bleibt. Diazotierung bei -20° C bis 50° C in verd. Mineralsäuren führt zu α-Bromcarbonsäuren oder über die Milchsäuren zu α-Trifluormethansulfonyloxy-Carbonsäuren, die mit einem $R^1$ und $R^{10}$ tragenden Nucleophil umgesetzt werden können;

c) Formel IV fallen, werden ausgehend von Malonestern hergestellt, deren Alkylierung mit Arylalkylhalogeniden mono- oder disubstituierte Malonester liefert, die nach Verseifung durch Decarboxylierung in die gewünschten Derivate überführt werden. Für den Fall, daß einer der Reste $R^{11}$ oder $R^{12}$ Hydroxy bedeutet, geht man von der entsprechenden Aminosäure aus und erhält nach Diazotierung (wie oben beschrieben) die Milchsäure (Anzahl der $R^{11}$ oder $R^{12}$ tragenden $CH_2$-Gruppen = 0) oder man geht von der substituierten Malonsäure aus, wobei Monoverseifung und selektive Reduktion (mit z.B. Diboran oder $LiAlH_4$) die 2-substituierte 3-Hydroxy-propionsäure ergibt.

Fragmente der Formel VII werden nach den allgemeinen bekannten Methoden zur Herstellung von Aminosäuren und Peptiden synthetisiert.

Zur Synthese der Fragmente der Formel X werden N-geschützte α-Aminosäuren nach B. Castro et al. (Synthesis 1983, 676) in die α-Aminoaldehyde überführt. Dann erfolgt Wittig-Reaktion mit einem den Rest $R^5$ tragenden Phosphoniumsalz und anschließend eine Hydroborierung.

Die zur Herstellung von Verbindungen der Formel I erforderlichen Vor- und Nachoperationen wie Einführung und Abspaltung von Schutzgruppen sind literaturbekannt und sind z.B. in T.W. Greene "Protective Groups in Organic Synthesis" (John Wiley & Sons, New York, 1981) beschrieben. Salze von Verbindungen der Formel I mit salzbildenden Gruppen werden in an sich bekannter Weise hergestellt,

indem man z.B. eine Verbindung der Formel I mit einer basischen Gruppe mit einer stöchiometrischen Menge einer geeigneten Säure oder Verbindungen der Formel I mit einer sauren Gruppe mit einer stöchiometrischen Menge einer geeigneten Base umsetzt. Stereoisomerengemische, insbesondere Diastereomerengemische, die gegebenenfalls bei der Synthese von Verbindungen der Formel I anfallen, können in an sich bekannter Weise durch fraktionierte Kristallisation oder durch Chromatographie getrennt werden.

Die erfindungsgemäßen Verbindungen der Formel I weisen enzymhemmende Eigenschaften auf, insbesondere hemmen sie Aspartylproteasen wie das Renin.

Renin wird als Folge verschiedener Stimuli (Volumendepletion, Natriummangel, β-Rezeptorenstimulation) von den juxtaglomerulären Zellen der Niere in den Blutkreislauf sezerniert. Dort spaltet es von dem aus der Leber ausgeschiedenen Angiotensinogen das Decapeptid Angiotensin I ab. Dieses wird durch das "angiotensin converting enzyme" (ACE) in Angiotensin II überführt. Angiotensin II spielt eine wesentliche Rolle bei der Blutdruckregulation, da es direkt den Blutdruck durch Gefäßkonstriktion steigert. Zusätzlich stimuliert es die Sekretion von Aldosteron aus der Nebenniere und erhöht auf diese Weise über die Hemmung der Natrium-Ausscheidung das extrazelluläre Flüssigkeitsvolumen, was seinerseits zu einer Blutdrucksteigerung beiträgt. Hemmer der enzymatischen Aktivität des Renins bewirken eine verminderte Bildung von Angiotensin I, was eine verminderte Bildung von Angiotensin II zur Folge hat. Die Erniedrigung der Konzentration dieses aktiven Peptidhormons ist die direkte Ursache für die blutdrucksenkende Wirkung von Renin-Hemmern.

Die Wirksamkeit von Renin-Hemmern kann durch in-vitro-Tests überprüft werden. Hierbei wird die Verminderung der Bildung von Angiotensin I in verschiedenen Systemen (Humanplasma, gereinigtes Humanrenin) gemessen.

**1. Testprinzip**

Z.B. Humanplasma, welches sowohl Renin als auch Angiotensinogen enthält, wird bei 37° C mit der zu testenden Verbindung inkubiert. Dabei wird aus Angiotensinogen unter der Einwirkung von Renin Angiotensin I freigesetzt, das anschließend mit einem handelsüblichen Radioimmunoassay gemessen werden kann. Diese Angiotensin-Freisetzung wird durch Renin-Inhibitoren gehemmt.

**2. Gewinnung des Plasmas**

Das Blut wird von freiwilligen Probanden gewonnen (ca. 0,5 l pro Person; Bluko-Entnahmegerät der Fa. ASID Bonz und Sohn, Unterschleißheim) und in teilweise evakuierten Flaschen unter Eiskühlung aufgefangen. Die Gerinnung wird durch Zugabe von EDTA (Endkonzentration 10 mM) verhindert. Nach dem Zentrifugieren (Rotor HS 4 (Sorvall), 3 500 Upm, 0-4° C, 15 min; wiederholen, falls erforderlich) wird das Plasma vorsichtig abpipettiert und in geeigneten Portionen bei -30° C eingefroren. Für den Test werden nur Plasmen mit ausreichend hoher Reninaktivität verwendet. Plasmen mit niedriger Reninaktivität werden durch eine Kältebehandlung (-4° C, 3 Tage) aktiviert (Prorenin → Renin).

**3. Durchführung des Tests**

Angiotensin I wird mit dem Renin-Maia®-Kit (Serono Diagnostics S.A., Coinsins, Schweiz) bestimmt. Die Inkubation des Plasmas wird nach der dort angegebenen Anleitung durchgeführt:

Inkubationsansatz: 1000 µl Plasma (bei 0-4° C aufgetaut)
100 µl Phosphatpuffer (pH 7,4)
Zusatz von $10^{-4}$ M Ramiprilat)
10 µl PMSF-Lösung
10 µl 0,1 % Genapol PFIC
12 µl DMSO bzw. Testpräparat

Die Testpräparate werden i.a. $10^{-2}$ M in 100 % Dimethylsulfoxid (DMSO) gelöst und mit DMSO entsprechend verdünnt; der Inkubationsansatz enthält max. 1 % DMSO.

Die Ansätze werden in Eis gemischt und für 1 Stunde zur Inkubation in ein Wasserbad (37° C) gestellt. Aus einem zusätzlichen Ansatz ohne Inhibitor werden ohne weitere Inkubation insgesamt 6 Proben (jeweils 100 µl) zur Bestimmung des Ausgangs-Angiotensin I-Gehaltes des verwendeten Plasmas entnommen.

Die Konzentrationen der Testpräparate werden so gewählt, daß etwa der Bereich von 10-90 %

Enzymhemmung abgedeckt ist (mindestens fünf Konzentrationen). Am Ende der Inkubationszeit werden aus jedem Ansatz drei 100 µl-Proben in vorgekühlten Eppendorf-Gefäßen auf Trockeneis eingefroren und bei ca. -25° C für die Angiotensin I-Bestimmung aufbewahrt (Mittelwert aus drei Einzelproben).

**Angiotensin I-Radioimmunoassay (RIA)**

Es wird exakt die Gebrauchsanweisung des RIA-Kits (Renin-Maia®-Kit, Serono Diagnostics S.A., Coinsins, Schweiz) befolgt.

Die Eichkurve umfaßt den Bereich von 0,2 bis 25,0 ng Angiotensin I pro ml. Der Basis-Angiotensin I-Gehalt des Plasmas wird von allen Meßwerten abgezogen. Die Plasma-Renin-Aktivität (PRA) wird als ng Ang I/ml x Stunde angegeben. PRA-Werte in Gegenwart der Testsubstanzen werden auf einen Ansatz ohne Inhibitor (= 100 %) bezogen und als % Restaktivität angegeben. Aus der Auftragung von % Restaktivität gegen die Konzentration (M) des Testpräparates (logarithmische Skala) wird der $IC_{50}$-Wert abgelesen.

Die in der vorliegenden Erfindung beschriebenen Verbindungen der allgemeinen Formel I zeigen in dem in-vitro-Test Hemmwirkungen bei Konzentrationen von etwa $10^{-5}$ bis $10^{-10}$ Mol/l.

Renin-Hemmer bewirken an salzverarmten Tieren eine Blutdrucksenkung. Da sich menschliches Renin von dem Renin anderer Spezies unterscheidet, werden zum in-vivo-Test von Renin-Hemmern Primaten, wie zum Beispiel Rhesus-Affen, herangezogen. Primaten-Renin und Human-Renin sind in ihrer Sequenz weitgehend homolog. Durch i.v. Injektion von Furosemid wird eine endogene Renin-Ausschüttung angeregt. Anschließend werden die Testverbindungen verabreicht und ihre Wirkung auf Blutdruck und Herzfrequenz wird gemessen. Die Verbindungen der vorliegenden Erfindung sind hierbei in einem Dosisbereich von etwa 0,1 - 5 mg/kg i.v. wirksam, bei intraduodenaler Applikation per Gastroskop im Dosisbereich von etwa 1 - 50 mg/kg. Die in der vorliegenden Erfindung beschriebenen Verbindungen der allgemeinen Formel I können als Antihypertensiva sowie zur Behandlung der Herzinsuffizienz verwendet werden.

Zum Gegenstand der Erfindung gehört weiterhin die Verwendung von Verbindungen der Formel I zur Herstellung von Arzneimittel zur Bluthochdrucktherapie und der Behandlung der kongestiven Herzinsuffizienz sowie die genannten Arzneimittel.

Pharmazeutische Präparate enthalten eine wirksame Menge des Wirkstoffs der Formel I zusammen mit einem anorganischen oder organischen pharmazeutisch verwendbaren Trägerstoff.

Die Anwendung kann intranasal, intravenös, subkutan, peroral oder intraduodenal erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier-oder Dragierverfahren hergestellt.

Für die orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- als auch als Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösungen, Suspensionen oder Emulsionen gebracht. Als Lösungsmittel kommen z.B. in Frage: Wasser, physiologische Kochsalzlösungen oder Alkohole, z.B. Ethanol, Propandiol oder Glycerin, daneben auch Zuckerlösungen wie Glucose-oder Mannitlösungen, oder auch eine Mischung aus den verschiedenen genannten Lösungsmitteln.

**Verzeichnis der verwendeten Abkürzungen:**

| | |
|---|---|
| Ac | Acetyl |
| Boc | tert.-Butoxycarbonyl |
| BuLi | n-Butyllithium |
| DC | Dünnschichtchromatographie |
| DCC | Dicyclohexylcarbodiimid |
| DCI | Desorption Chemical Ionisation |
| DIP | Diisopropylether |
| DNP | 2,4-Dinitrophenyl |
| DMF | Dimethylformamid |
| DMSO | Dimethylsulfoxid |
| EE | Essigsäureethylester |
| EI | Electron Impact |
| Etoc | Ethoxycarbonyl |
| FAB | Fast atom bombardment |
| H | Hexan |
| HOBt | 1-Hydroxybenzotriazol |
| Iva | Isovaleryl |
| M | Molekularpeak |
| MeOH | Methanol |
| MS | Massenspektrum |
| MTB | Methyl-tert.-butylether |
| Nva | Norvalin |
| Nle | Norleucin |
| R.T. | Raumtemperatur |
| Schmp. | Schmelzpunkt |
| $Sdp_{xx}$ | Siedepunkt bei xx Torr |
| Thi | $\beta$-2-Thienylalanin |
| THF | Tetrahydrofuran |
| Z | Benzyloxycarbonyl. |

Die sonstigen für Aminosäuren verwendeten Abkürzungen entsprechen dem in der Peptidchemie üblichem Drei-Buchstaben-Code wie er z.B. in Eur. J. Biochem. 138, 9 - 37 (1984) beschrieben ist. Falls nicht ausdrücklich anders angegeben, handelt es sich immer um Aminosäuren der L-Konfiguration.

Die nachstehenden Beispiele dienen zur Erläuterung der vorliegenden Erfindung, ohne daß diese darauf beschränkt wäre.

## Beispiel 1

[3-tert.-Butylsulfonyl, 2-(2)-thienylmethyl]propionyl-Nva-[1-cyclohexylmethyl,2-(4,4,5,5-tetramethyl-1,3,2-dioxaborolan-2-yl),5-methyl]hexylamid

346 mg [3-tert.-Butylsulfonyl,2-(2)-thienylmethyl]-propionyl-Nva-OH werden in 40 ml THF gelöst und bei -20 °C zunächst 98 µl N-Methylmorpholin, dann 115 µl Chlorameisensäureisobutylester zugespritzt. Nach 10 Minuten bei -20 °C wird 123 µl Triethylamin addiert. Das so erhaltene gemischte Anhydrid wird bei -20 °C zu einer Lösung von 400 mg [1-Cyclohexylmethyl,2-(4,4,5,5-tetramethyl-1,3,2-dioxaborolan-2-yl),5-methyl]hexylammonium-trifluoracetat in 40 ml THF zugespritzt. Es wird 1 h bei -20 °C gerührt und 22 h bei R.T. stehen gelassen. Man verdünnt mit 100 ml MTB, wäscht 2x mit je 30 ml 5 % wäßriger $NaHSO_4$-Lösung und 2x mit je 30 ml 5 % wäßriger $Na_2CO_3$-Lösung. Die organische Phase wird über $Na_2SO_4$ getrocknet und das Solvens im Vakuum entfernt. Nach Chromatographie an Kieselgel mit MTB/DIP = 1:1 erhält man 160 mg der Titelverbindung als farblose Kristalle.

$R_f$ (MTB/DIP 1:1) = 0,50 MS (FAB):709 (M+1)

a) [3-t-Butylsulfonyl,2-(2-thienylmethyl)]propionyl-Nva-OH

490 mg [3-t-Butylsulfonyl,2-(2-thienylmethyl)]propionyl-Nva-OMe werden in 4 ml Methanol gelöst, 0,4 ml $H_2O$ sowie 0,7 ml 2N NaOH zugegeben und 5h bei R.T. gerührt. Mit $NaHSO_4$-Lösung wird auf pH = 2 angesäuert und 3x mit 50 ml EE extrahiert. Anschließend wird über $Na_2SO_4$ getrocknet und das Solvens im Vakuum entfernt. Man erhält 470 mg blaßgelbes Harz.
$R_f$ (EE) = 0,24 MS (FAB): 390 (M + 1)

b) [3-t-Butylsulfonyl,2-(2-thienylmethyl)]propionyl-Nva-OMe

1,5 g [3-t-Butylsulfonyl,2-(2-thienylmethyl)]propionsäure sowie 0,95 g Nva-OMe x HCl werden in 40 ml $CH_2Cl_2$ gelöst und bei 10 °C zunächst 3,8 ml Triethylamin, dann 3,5 ml einer 50 % Lösung von Propanphosphonsäure-Anhydrid in $CH_2Cl_2$ zugegeben. 20 h wird bei R.T. gerührt, das Solvens im Vakuum entfernt, in 100 ml MTB aufgenommen und mit je 100 ml $NaHSO_4$-Lösung und $NaHCO_3$-Lösung gewaschen. Über $Na_2SO_4$ wird getrocknet, das Solvens im Vakuum entfernt und mit EE/H 1:1 chromatographiert. Man erhält 2 diastereomere Öle, die getrennt weiter verarbeitet werden.

| | |
|---|---|
| Diastereomer 1 $R_f$ (EE/H 1:1) = 0,30 | 0,95 g |
| Diastereomer 2 $R_f$ (EE/H 1:1) = 0,20 | 0,95 g |

MS (DCI, für beide Diastereomere gleich): 404 (M + 1)

c) [3-t-Butylsulfonyl,2-(2-thienylmethyl)]propionsäure

4,4 g [3-t-Butylsulfonyl,2-(2-thienylmethyl)]propionsäuremethylester werden in 50 ml 5N HCl suspendiert und 2 h zum Rückfluß erhitzt. Nach Abkühlung wird 3x mit 50 ml EE extrahiert, über $Na_2SO_4$ getrocknet und das Solvens im Vakuum entfernt. Man erhält 4,0 g der Titelverbindung als blaßgelbes Öl.
$R_f$ (MTB) = 0,15 - 0,25 MS (DCI): 291 (M + 1)

d) [3-t-Butylsulfonyl,2-(2-thienylmethyl)]propionsäuremethylester

8,4 g [3-t-Butylthio,2-(2-thienylmethyl)]propionsäuremethylester werden in 100 ml $CH_2Cl_2$ gelöst und 10,6 g m-Chlorperbenzoesäure unter Eiskühlung portionsweise zugegeben. 1 h wird bei R.T. gerührt, anschließend zunächst mit 100 ml 10 % $Na_2SO_3$, dann mit 100 ml $NaHCO_3$ gewaschen. Über $Na_2SO_4$ wird getrocknet und das Solvens im Vakuum entfernt. Man erhält 7,2 g der Titelverbindung als farbloses Öl.
$R_f$ (MTB) = 0,56 MS (DCI): 305 (M + 1)

e) [3-t-Butylthio,2-(2-thienylmethyl)]propionsäuremethylester

6,1 ml t-Butylmercaptan werden in 100 ml MeOH (wasserfrei) gelöst und unter Argon 130 mg NaH zugegeben. Anschließend werden 7,6 g 2-(2-Thienylmethyl)acrylsäure-methylester zugetropft und 4 h bei R.T. gerührt. Das Solvens wird im Vakuum entfernt, in 100 ml MTB aufgenommen und mit 100 ml 5 % $NaHSO_4$-Lösung gewaschen. Über $Na_2SO_4$ wird getrocknet und das Solvens im Vakuum entfernt. Man erhält 10,4 g der Titelverbindung als blaßgelbe Flüssigkeit, die ohne Reinigung und Charakterisierung weiter eingesetzt wird.
$R_f$ (DIP/H 1:5) = 0,31

f) 2-(2-Thienylmethyl)acrylsäure-methylester

11,8 g 2-Thienylmethylmalonsäure-monomethylester, 5,8 ml Diethylamin und 5,0 ml 36 % wäßrige Formaldehyd-Lösung werden bei R.T. unter Argon 1 h gerührt. Das Wasser wird anschließend im Vakuum entfernt und der Rückstand chromatographiert. Man erhält 7,6 g der Titelverbindung als farblose Flüssigkeit.
$R_f$ (MTB/H 1:5) = 0,49

g) 2-Thienylmethylmalonsäure-monomethylester

20,1 g 2-Thienylmethylmalonsäure-dimethylester werden in 300 ml MeOH gelöst und 5,0 g KOH zugegeben. Bei R.T. wird 7 h gerührt, das Solvens im Vakuum entfernt und mit je 100 ml 5 % $Na_2CO_3$-Lösung und EE aufgenommen. Anschließend wird die wäßrige Phase auf pH=2 angesäuert und 3 x mit je 100 ml EE extrahiert. Über $Na_2SO_4$ wird getrocknet und das Solvens im Vakuum entfernt. Man erhält 16,0 g der Titelverbindung als blaßgelbes Öl.
$R_f$ (EE/MeOH 6:1) = 0,3 - 0,4

h) 2-Thienylmethylmalonsäure-dimethylester

87,8 g Malonsäuredimethylester und 41,0 g Kalium-t-butylat werden unter Eiskühlung in 1,1 l THF (wasserfrei) gelöst und unter Argon 44,1 g 2-Thienylmethylchlorid in 500 ml THF zugetropft. 3 h wird bei R.T. gerührt, das KCl abfiltriert, das Solvens im Vakuum entfernt und der Rückstand chromatographiert. Man erhält 33,8 g der Titelverbindung (I) als farbloses Öl neben 8,8 g Bis-(2-Thienylmethyl)-malonsäuredimethylester (II)
$R_f$ (I) (Toluol/DIP 20:1) = 0,35
$R_f$ (II) (Toluol/DIP 20:1) = 0,44

g) 2-Thienylmethylchlorid

252 g Thiophen werden in 128 ml konzentrierter wäßriger HCl suspendiert und bei 0° C 1 h lang HCl-Gas eingeleitet. Anschließend werden ohne Unterbrechung des HCl-Stromes 255 ml 35 % wäßrige Formaldehydlösung zugetropft und weitere 15 Minuten bei 0° C gerührt. Nach Abtrennung der organischen Phase wird die wäßrige Phase noch 2x mit 600 ml $CH_2Cl_2$ extrahiert. Anschließend wird 2x mit 600 ml gesättigter wäßriger $Na_2CO_3$ gewaschen, über $Na_2SO_4$ getrocknet, das Solvens im Vakuum entfernt und destilliert. Man erhält 174 g der Titelverbindung als farblose Flüssigkeit.
$Sdp_{22}$ = 81 - 84° C

h) [1-Cyclohexylmethyl,2-(4,4,5,5-tetramethyl-1,3,2-dioxaborolan-2-yl),5-methyl]hexylammonium-trifluoracetat

520 mg Z-[1-Cyclohexylmethyl,2-(4,4,5,5-tetramethyl-1,3,2-dioxaborolan-2-yl),5-methyl]hexylamin und 95 µl Trifluoressigsäure werden in 10 ml EtOH gelöst, mit 105 mg Pd/C versetzt und 2,5 h bei R.T. unter 1 bar Druck hydriert. Anschließend wird der Katalysator abfiltriert und das Solvens im Vakuum entfernt. Man erhält 400 mg der Titelverbindung als farbloses Öl, das ohne Reinigung weiter eingesetzt wird.
MS (DCI) : 338 (M+1)

i) Z-[1-Cyclohexylmethyl,2-(4,4,5,5-tetramethyl-1,3,2-dioxaborolan-2-yl),5-methyl]hexylamin

6,5 g Z-(1-Cyclohexylmethyl,5-methyl)-hex-2-en-amin werden in 190 ml THF unter Argon gelöst. Bei 0° C wird 5,7 ml $BH_3.S(CH_3)_2$ zugetropft und 1 h bei R.T. gerührt. Anschließend wird langsam Wasser zugetropft bis die Wasserstoffentwicklung beendet ist. Das Solvens wird im Vakuum entfernt und 2x mit je 100 ml Toluol nachrotiert. Der Rückstand wird in 120 ml $CH_2Cl_2$ gelöst und 11,2 g Pinakol zugegeben. 4 h wird am Wasserabscheider gekocht, das $CH_2Cl_2$ im Vakuum entfernt und an Kieselgel mit EE/n-Hexan = 1:8 chromatographiert. Man erhält 4,7 g der Titelverbindung als Diastereomerengemisch, das durch erneute Chromatograpie getrennt werden kann.
$R_f$ (EE/n-Hexan = 1:8) = 0,25 0,22 MS (DCI) : 472 (M+1)

k) Z-(1-Cyclohexylmethyl,5-methyl)-hex-2-en-amin

13,1 g (Isopentyl, triphenyl)phosphoniumbromid werden in 280 ml THF suspendiert und mit 3,2 g Kalium-tert.-Butylat versetzt. 2,5 h wird bei R.T. gerührt, dann auf 0° C gekühlt und 8,3 g Z-(2(S)-Cyclohexylmethyl)-aminoacetaldehyd in 100 ml THF zugetropft. 1 h wird bei R.T. gerührt, mit 200 ml MTB

versetzt und 2x mit je 50 ml 5 % wäßriger $NaHSO_4$-Lösung und 2x mit je 50 ml 5 % wäßriger $NaHCO_3$-Lösung gewaschen. Die organische Phase wird über $Na_2SO_4$ getrocknet und das Solvens im Vakuum entfernt. Nach Chromatographie an Kieselgel erhält man 6,5 g der Titelverbindung als farbloses Öl.
$R_f$ (EE/n-Hexan = 1:8) = 0,28 MS (DCI) : 344 (M+1)
Beispiele 2 und 3 wurden analog Beispiel 1 synthetisiert:

**Beispiel 2**

[2-(S)-Benzyl,3-tert.-butylsulfonyl]propionyl-Nva-[1-cyclohexylmethyl,2-(4,4,5,5-tetramethyl-1,3,2-dioxaborolan-2-yl),5-methyl]hexylamid

$R_f$ (MTB/DIP 1:1) = 0,52 MS (FAB) : 703 (M+1)

**Beispiel 3**

[3-tert.-Butylsulfonyl,2-(1-naphthylmethyl)]propionyl-Val-[1-cyclohexylmethyl,2-(4,4,5,5-tetramethyl-1,3,2-dioxaborolan-2-yl),2-cyclopentyl]methylamid

$R_f$ (MTB/DIP 1:1) = 0,42 MS (FAB) : 751 (M+1)

**Beispiel 4**

Boc-Phe-His-[1-Cyclohexylmethyl,2-(4,4,5,5-tetramethyl-1,3,2-dioxaborolan-2-yl),5-methyl]hexylamid

300 mg Boc-Phe-His(DNP)-[1-Cyclohexylmethyl,2-(4,4,5,5-tetramethyl-1,3,2-dioxaborolan-2-yl),5-methyl]-hexylamid werden in 5 ml Acetonitril gelöst und mit 281 µl Thiophenol versetzt. 3 h wird bei R.T. gerührt, das Solvens i. Vak. entfernt und an Kieselgel mit MeOH/EE 1:20 chromatographiert. Man erhält 130 mg der Titelverbindung als farbloses amorphes Pulver.
$R_f$ (EE/MeOH 20:1) = 0,35 MS (FAB) : 722 (M+1)

a) Boc-Phe-His(DNP)-[1-Cyclohexylmethyl,2-(4,4,5,5-tetramethyl-1,3,2-dioxaborolan-2-yl),5-methyl]hexylamid

Die Titelverbindung wurde analog Beispiel 1 synthetisiert.
Rf (MTB) = 0,27 MS (FAB) : 872 (M+1)
Beispiel 5 wurde analog Beispiel 4 synthetisiert:

**Beispiel 5**

[2-(S)-Benzyl,3-tert.-butylsulfonyl]propionyl-His-[1-cyclohexylmethyl,2-(4,4,5,5-tetramethyl-1,3,2-dioxaborolan-2-yl),5-methyl]hexylamid

$R_f$ (EE/MeOH 10:1) = 0,20 MS (FAB) : 741 (M+1)

**Beispiel 6**

[2-(S)-Benzyl,3-tert.-butylsulfonyl]propionyl-His-[1-cyclohexylmethyl,2-dihydroxyboryl,5-methyl]hexylamid

217 mg der Titelverbindung des Beispiels 5 und 220 µl Titanisopropoxid werden in 100 ml n-Propanol gelöst und 8 h gekocht an einem Soxhlet-Extraktor, der mit Molekularsieb 0,3 nm gefüllt ist. Die Reaktionslösung wird auf 100 ml ges. $NaHCO_3$-Lösung gegossen, das $TiO_2$ abfiltriert, 3 mal mit 50 ml EE

extrahiert und über $Na_2SO_4$ getrocknet. Das Solvens wird i. Vak. entfernt und an Kieselgel mit $CH_2Cl_2$/MeOH 10:1 chromatographiert. Man erhält 43 mg der Titelverbindung als weißes amorphes Pulver.
$R_f$ ($CH_2Cl_2$/MeOH 10:1) = 0,28 MS (FAB) : 659 (M+1)

## Ansprüche

1. Verbindung der Formel I

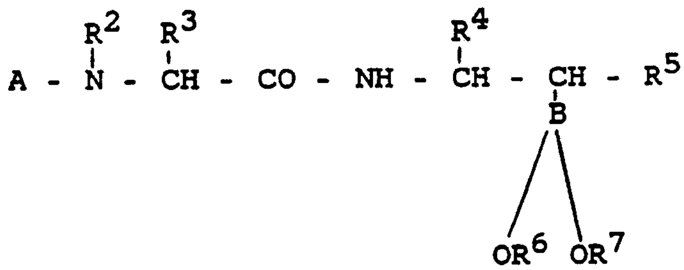

(I)

in welcher
A einen Rest der Formeln II, III oder IV bedeutet

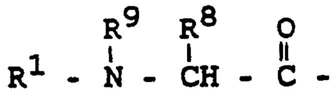

(II)

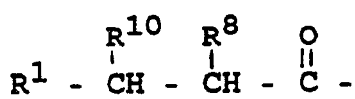

(III)

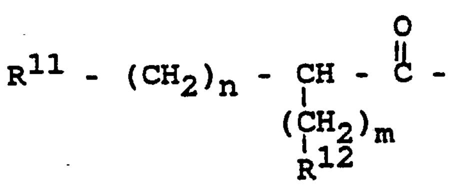

(IV)

worin
$R^1$ $a_1$) Wasserstoff, ($C_1$-$C_{12}$)-Alkyl, das gegebenenfalls ein-oder zweifach ungesättigt ist und das gegebenenfalls durch bis zu 3 gleiche oder verschiedene Reste aus der Reihe Hydroxy, ($C_1$-$C_7$)-Alkoxy, Carbamoyl, ($C_1$-$C_8$)-Alkanoyloxy, Carboxy, ($C_1$-$C_7$)-Alkoxycarbonyl, F, Cl, Br, I, Amino, Amidino, das gegebenenfalls durch einen, zwei oder drei ($C_1$-$C_8$)-Alkylreste substituiert sein kann, Guanidino, das gegebenenfalls durch einen, zwei, drei oder vier ($C_1$-$C_8$)-Alkylreste substituiert sein kann, ($C_1$-$C_7$)-Alkylamino, Di-($C_1$-$C_7$)-alkylamino, ($C_1$-$C_5$)-Alkoxycarbonylamino, ($C_7$-$C_{15}$)-Aralkoxycarbonylamino und 9-Fluorenylmethoxycarbonylamino substituiert ist; mono-, bi- oder tricyclisches ($C_3$-$C_{18}$)Cycloalkyl, ($C_3$-$C_{18}$)-Cycloalkyl-($C_1$-$C_6$)-alkyl oder ($C_6$-$C_{14}$)-Aryl, wobei der Cycloalkylteil gegebenenfalls durch ($C_1$-$C_6$)-Alkyl substituiert ist und Aryl gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino und Trifluormethyl substituiert ist;
($C_6$-$C_{14}$)-Aryl-($C_1$-$C_6$)-alkyl, worin der Arylteil gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino, ($C_1$-$C_7$)-Alkylamino, Di-($C_1$-$C_7$)-alkylamino, Carboxy, Carboxymethoxy, Amino-($C_1$-$C_7$)-alkyl, ($C_1$-$C_7$)-Alkylamino-($C_1$-$C_7$)-alkyl, Di-($C_1$-$C_7$)-alkylamino-($C_1$-$C_7$)-alkyl, ($C_1$-$C_7$)-Alkoxycarbonylmethoxy, Carbamoyl, Sulfamoyl, ($C_1$-$C_7$)-Alkoxysulfonyl, Sulfo- und Guanidino-($C_1$-$C_8$)-alkyl substituiert ist; oder für den Rest

eines 5- oder 6-gliedrigen monocyclischen oder 9-oder 10-gliedrigen bicyclischen teilweise oder vollständig hydrierten Heteroaromaten, mit mindestens 1 C-Atom, 1 - 4 N-Atomen und/oder 1 - 2 S-Atomen und/oder 1 - 2 O-Atomen als Ringglieder steht, und der gegebenenfalls wie ($C_6$-$C_{14}$)-Aryl unter $a_1$) definiert, mono-, di- oder trisubstituiert ist, bedeutet

oder

$a_2$) einen Rest der Formel V bedeutet

$R^1$ - W , (V)

worin $R^1$ wie $R^1$ unter $a_1$) definiert ist und W für

-CO-, -O-CO-, $-SO_2-$, -SO-, $-NH-SO_2-$, -NH-CO-, -CH(OH)-oder -N(OH)- steht;

$R^2$ Wasserstoff oder ($C_1$-$C_8$)-Alkyl bedeutet, oder zusammen mit $R^3$ und den diese tragenden Atomen ein mono- oder bicyclisches, gesättigtes oder teilweise ungesättigtes Ringsystem mit 5-12 Ringgliedern bildet;

$R^3$ und $R^8$ unabhängig voneinander wie $R^1$ und $a_1$) definiert sind oder

zusammen mit $R^2$ bzw. mit $R^9$ und den diese tragenden Atomen Ringsysteme mit 5-12 Ringgliedern bilden wie oben definiert;

$R^4$ ($C_3$-$C_{12}$)-Alkyl, mono-, bi- oder tricyclisches ($C_3$-$C_{18}$)-Cycloalkyl, ($C_3$-$C_{18}$)-Cycloalkylmethyl, ($C_3$-$C_{18}$)-Cycloalkylethyl, wobei der Cycloalkylteil gegebenenfalls durch ($C_1$-$C_6$)-Alkyl substiuiert ist; Dithiolanyl; ($C_6$-$C_{14}$)-Arylmethyl; Dithiolanylmethyl; Dithiolanylethyl; Dithianyl; Dithianylmethyl oder Dithianylethyl bedeutet;

$R^5$ Wasserstoff; Azido; Azido-($C_1$-$C_4$)-alkyl; ($C_1$-$C_{12}$)-Alkyl, das gegebenenfalls durch Hydroxy, Azido oder Hydroxy und Azido substituiert sein kann; ($C_3$-$C_{12}$)-Cycloalkyl; ($C_3$-$C_{12}$)-Cycloalkyl-($C_1$-$C_6$)-alkyl; $C_3$-$C_{12}$)-Cycloalkylsulfonyl-($C_1$-$C_6$)-alkyl; ($C_1$-$C_6$)-Alkylsulfonyl-($C_1$-$C_6$)-alkyl; ($C_6$-$C_{14}$)-Aryl, ($C_6$-$C_{14}$)-Aryl-($C_1$-$C_6$)-alkyl, wobei Aryl auch für den Rest eines 5- oder 6-gliedrigen monocyclischen oder 9-oder 10-gliedrigen bicyclischen Heteraromaten mit mindestens 1 C-Atom, 1-4 N-Atomen und/oder 1-2 S-Atomen und/oder 1-2 O-Atomen als Ringglieder stehen kann, der auch teilweise oder vollständig hydriert sein kann, ebenfalls ist es möglich daß Aryl und Heteroaryl wie unter $a_1$) definiert substituiert sein kann,

$R^6$ und $R^7$ unabhängig voneinander Wasserstoff oder ($C_1$-$C_6$)-Alkyl bedeuten; oder

zusammen mit dem Boratom und Sauerstoffatomen ein mono-, bi- oder tricyclisches, gesättigtes oder teilweise ungesättigtes, mono-, di-, tri- oder tetra-($C_1$-$C_6$)-alkyliertes oder -phenyliertes Ringsystem mit 5-18 Ringgliedern, das außer dem Boratom, den Sauerstoffatomen und dem/den Kohlenstoffatom(en) noch ein -O- Glied, ein $-NR^{13}$-Glied oder ein $-CR^{14}R^{15}$-Glied enthalten kann, bilden;

$R^9$ für Wasserstoff oder ($C_1$-$C_8$)-Alkyl steht, oder zusammen mit $R^8$ und den diese tragenden Atomen ein mono- oder bicyclisches, gesättigtes oder teilweise ungesättigtes Ringsystem mit 5-12 Ringgliedern bildet;

$R^{10}$ Wasserstoff oder ($C_1$-$C_8$)-Alkyl ist, oder zusammen mit $R^1$ oder $R^8$ und den diese tragenden Atomen ein mono- oder bicyclisches, gesättigtes oder teilweise ungesättigtes Ringsystem mit 5-12 Ringgliedern bildet, das außer Kohlenstoff noch 1 Schwefelatom enthalten kann, welches gegebenenfalls zum Sulfoxid oder Sulfon oxidiert sein kann;

$R^{11}$ und $R^{12}$ unabhängig voneinander Wasserstoff, Hydroxy oder ($C_6$-$C_{14}$)-Aryl bedeuten, wobei Aryl gegebenenfalls durch einen oder zwei gleiche oder verschiedene Reste aus der Reihe F, Cl, Br, I,.Hydroxy, ($C_1$-$C_7$)-Alkoxy, ($C_1$-$C_7$)-Alkyl, ($C_1$-$C_7$)-Alkoxycarbonyl, Amino, ($C_1$-$C_7$)-Alkylamino, Di-($C_1$-$C_7$)-alkylamino, Carboxy, Carboxymethoxy, Amino-($C_1$-$C_7$)-alkyl, ($C_1$-$C_7$)-Alkylamino-($C_1$-$C_7$)-alkyl, Di-($C_1$-$C_7$)-alkylamino-( $C_1$-$C_7$)-alkyl, ($C_1$-$C_7$)-Alkoxycarbonylmethoxy, Carbamoyl, Sulfamoyl, ($C_1$-$C_7$)-Alkoxysulfonyl, Sulfo- und Guanidino-($C_1$-$C_8$)-alkyl substituiert ist; oder für den Rest eines 5- oder 6-gliedrigen monocyclischen oder 9- oder 10-gliedrigen bicyclischen, gegebenenfalls teilweise oder vollständig hydrierten Heteroaromaten, mit mindestens 1 C-Atom, 1 - 4 N-Atomen und/oder 1 - 2 S-Atomen und/oder 1 - 2 O-Atomen als Ringglieder stehen, der gegebenenfalls wie oben ($C_6$-$C_{14}$)-Aryl mono- oder disubstituiert ist,

n und m unabhängig voneinander 0, 1, 2, 3 und 4 sein können,

$R^{13}$ Wasserstoff oder ($C_1$-$C_{12}$)-Alkyl, das gegebenenfalls ein- bis zweifach ungesättigt ist und das gegebenenfalls durch bis zu 3 gleiche oder verschiedene Reste aus der Reihe Hydroxy, ($C_1$-$C_7$)-Alkoxy, Amino, Mono- oder Di-($C_1$-$C_7$)-alkylamino substituiert ist, bedeutet,

$R^{14}$ und $R^{15}$ unabhängig voneinander Wasserstoff, ($C_1$-$C_8$)-Alkyl, Hydroxymethyl, 2-Hydroxyethyl, (3-Hydroxysulfonyl, 2-hydroxypropyl)amino, (2-Hydroxysulfonylethyl)amino, (2-Hydroxysulfonylpropyl)amino, (Carboxymethyl)amino, Bis(2-hydroxyethyl)amino bedeuten

sowie deren physiologisch verträgliche Salze.

2. Verbindung der Formel I, gemäß Anspruch 1, dadurch gekennzeichnet, daß

$R^1$ Wasserstoff bedeutet oder für ($C_1$-$C_{10}$)-Alkyl;

Cyclopentyl; Cyclohexyl; Cyclopentyl-($C_1$-$C_{10}$)-alkyl; Cyclohexyl-($C_1$-$C_{10}$)-alkyl; gegebenenfalls substituiertes Phenyl-($C_1$-$C_8$)-alkyl; 2-Pyridyl-($C_1$-$C_8$)-alkyl; 3-Pyridyl-($C_1$-$C_8$)-alkyl; 4-Pyridyl-($C_1$-$C_8$)-alkyl; $H_2N$-($C_1$-$C_{10}$)-Alkyl; HO-($C_1$-$C_{10}$)-Alkyl; ($C_1$-$C_4$)-Alkoxy-($C_1$-$C_{10}$)-alkyl; ($C_1$-$C_4$)-Alkoxycarbonyl-($C_1$-$C_{10}$)-alkyl; ($C_1$-$C_8$)-Alkylsulfonyl; ($C_1$-$C_8$)-Alkylsulfinyl; ($C_1$- $C_8$)-Hydroxyalkylsulfonyl; ($C_1$-$C_8$)-Hydroxyalkylsulfinyl; Hydroxy-($C_1$-

$C_{10}$)-alkanoyl; ($C_1$-$C_8$)-Alkanoyloxy-($C_1$-$C_{10}$)-alkyl; ($C_1$-$C_{11}$)-Alkanoyl; gegebenenfalls geschütztes Amino-($C_1$-$C_{11}$)-alkanoyl; Di-($C_1$-$C_7$)-alkylamino-($C_2$-$C_{11}$)-alkanoyl; Piperidino-4-carbonyl; Morpholino-4-carbonyl; ($C_3$-$C_9$)-Cycloalkylcarbonyl; ($C_6$-$C_{10}$)-Aryl-($C_2$-$C_{11}$)-alkanoyl; 2-Pyridyl-($C_1$-$C_8$)-alkanoyl; 3-Pyridyl-($C_1$-$C_8$)-alkanoyl; 4-Pyridyl-($C_1$-$C_8$)-alkanoyl; gegebenenfalls durch Halogen, ($C_1$-$C_7$)-Alkyl, ($C_1$-$C_7$)-Alkoxy oder ($C_1$-$C_7$)-Alkoxycarbonyl substituiertes Benzoyl; Pyrrolyl-2-carbonyl, Pyridyl-3-carbonyl; Benzolsulfonyl; ($C_1$-$C_{10}$)-Alkoxycarbonyl; substituiertes ($C_1$-$C_{10}$)-Alkoxycarbonyl; ($C_6$-$C_{14}$)-Aryl-($C_1$-$C_6$)-alkoxycarbonyl steht, oder gemeinsam mit $R^{10}$ ein mono-oder bicyclisches, gesättigtes oder teilweise ungesättigtes Ringsystem mit 5-12 Ringgliedern, das außer Kohlenstoff noch 1 Schwefelatom enthalten kann, welches gegebenenfalls zum Sulfoxid oder Sulfon oxydiert sein kann,

$R^2$ Wasserstoff, Methyl oder Ethyl ist oder zusammen mit $R^3$ und den diese tragenden Atomen Pyrrolidin oder Piperidin bildet, die jeweils noch mit Cyclopentyl, Cyclohexyl oder Phenyl anneliert sein können,

$R^3$ und $R^8$ unabhängig voneinander Wasserstoff;

($C_1$-$C_{10}$)-Alkyl, daß gegebenenfalls ein- oder zweifach ungesättigt ist und das gegebenenfalls durch bis zu 3 gleiche oder verschiedene Reste aus der Reihe Hydroxy, ($C_1$-$C_7$)-Alkoxy, ($C_1$-$C_7$)-Alkanoyloxy, Carboxy, ($C_1$-$C_7$)-Alkoxycarbonyl, Cl, Br, Amino, Amidino, Guanidino, Carbamoyl, ($C_1$-$C_5$)-Alkoxycarbonylamino, ($C_6$-$C_{15}$)-Aralkoxycarbonylamino und 9-Fluorenylmethoxycarbonylamino substituiert ist; ($C_3$-$C_{12}$)-Cycloalkyl, ( $C_3$-$C_{12}$)-Cycloalkyl-($C_1$-$C_3$)-alkyl, mono- oder bicyclisches ($C_6$-$C_{14}$)-Aryl-($C_1$-$C_3$)-Alkyl, das gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino und Trifluormethyl substituiert ist, bedeutet oder für ($C_1$-$C_3$)-Alkyl, substituiert mit dem Rest eines 5- oder 6-gliedrigen monocyclischen oder 9- oder 10-gliedrigen bicyclischen, gegebenenfalls teilweise oder vollständig hydrierten Heteroatomen, mit mindestens 1 C-Atom, 1 - 4 N-Atomen und/oder 1 - 2 S-Atomen und/oder 1 - 2 O-Atomen als Ringglieder, der gegebenenfalls wie in Anspruch 1 für den Arylteil beschrieben mono- oder disubstituiert ist stehen, oder zusammen mit $R^2$ bzw. $R^9$ Ringsysteme, wie vorstehend unter $R^2$ beschrieben, bilden;

$R^4$ ($C_3$-$C_{12}$)-Alkyl; mono- bi- oder tricyclisches ($C_3$-$C_{18}$)-Cycloalkyl oder ($C_3$-$C_{18}$)-Cycloalkylmethyl, wobei der Cycloalkylteil gegebenenfalls durch ($C_1$-$C_4$)-Alkyl substituiert ist; ($C_6$-$C_{14}$)-Arylmethyl; Dithiolanyl; Dithiolanylmethyl; Dithianyl und Dithianylmethyl ist;

$R^5$ für Wasserstoff, Azido, Azido-($C_1$-$C_4$)-alkyl, ($C_1$-$C_{12}$)-Alkyl, ($C_3$-$C_{12}$)-Cycloalkyl, ($C_3$-$C_{12}$)-Cycloalkyl-($C_1$-$C_6$)-alkyl, (2-Pyridyl)-($C_1$-$C_4$)-alkyl, (3-Pyridyl)-($C_1$-$C_4$)-alkyl, (4-Pyridyl)-($C_1$-$C_4$)-alkyl, Imidazol-2-yl, Imidazol-1-yl, Imidazol-4-yl, (Imidazol-2-yl)-($C_1$-$C_4$)-alkyl, (Imidazol-1-yl)-($C_1$-$C_4$)-alkyl, (Imidazol-4-yl)-($C_1$-$C_4$)-alkyl, [1-($C_1$-$C_6$)-Alkylimidazol-2-yl]-($C_1$-$C_4$)-alkyl, (Imidazolin-2-yl)-($C_1$-$C_4$)-alkyl, [1-($C_1$-$C_6$)-Alkylimidazolin-2-yl]-($C_1$-$C_4$)-alkyl steht;

$R^6$ und $R^7$ unabhängig voneinander Wasserstoff sind oder zusammen mit dem Boratom und den Sauerstoffatomen ein mono-, bi- oder tricyclisches, gesättigtes oder teilweise ungesättigtes mono-, di-, tri- oder tetraalkyliertes oder -phenyliertes Ringsystem mit 5-18 Ringgliedern bilden, das außer dem Boratom, den Sauerstoffatomen und dem (den) Kohlenstoffatom(en) noch ein -O-Glied, ein -$NR^{13}$-Glied oder ein -$CR^{14}R^{15}$-Glied enthalten kann;

$R^9$ Wasserstoff, Methyl oder Ethyl ist oder zusammen mit $R^8$ und den diese tragenden Atomen Pyrrolidin oder Piperidin bilden, die jeweils zusätzlich mit Cyclopentyl, Cyclohexyl oder Phenyl anneliert sein können;

$R^{10}$ Wasserstoff oder Methyl ist, oder gemeinsam mit $R^1$ oder $R^8$ und den diese tragenden Atomen ein mono- oder bicyclisches gesättigtes oder teilweise ungesättigtes Ringsystem mit 5-12 Ringgliedern bilden, das außer Kohlenstoff noch 1 Schwefelatom enthalten kann, welches gegebenenfalls zum Sulfoxid oder Sulfon oxidiert sein kann,

$R^{11}$ und $R^{12}$ unabhängig voneinander Wasserstoff, Hydroxy, Phenyl, 2- oder 3-Thienyl, 2-, 3- oder 4-Pyridyl, 1-, 2-oder 4-Imidazolyl, 1- oder 2-Naphthyl; 2- oder 3-Benzo[b]thienyl sind;

n und m unabhängig voneinander 0, 1, 2, 3 und 4 bedeuten;

$R^{13}$ Wasserstoff, oder ($C_1$-$C_{12}$)-Alkyl ist,

$R^{14}$ und $R^{15}$ unabhängig voneinander Wasserstoff, Hydroxymethyl, 2-Hydroxyethyl, (3-Hydroxysulfonyl, 2-hydroxypropyl)amino, (2-Hydroxysulfonylethyl)amino, (2-Hydroxysulfonylpropyl)amino, (Carboxymethyl)-amino, Bis(2-hydroxyethyl)amino bedeuten,

sowie deren verträgliche Salze.

3. Verbindung der Formel I, gemäß Anspruch 1 oder 2, dadurch gekennzeichnet, daß

$R^1$ ($C_1$-$C_8$)-Alkylsulfonyl; ($C_1$-$C_8$)-Alkylsulfinyl; ($C_1$-$C_8$)-Hydroxyalkylsulfonyl; Hydroxy-($C_1$-$C_{10}$)-alkanoyl; ($C_1$-$C_8$)-Alkanoyloxy-($C_1$-$C_{10}$)-alkyl; ($C_1$-$C_{11}$)Alkanoyl; Amino-($C_1$-$C_{11}$)-alkanoyl; Di-($C_1$-$C_7$)-alkylamino-($C_2$-$C_{11}$)-alkanoyl; Piperidino-4-carbonyl; Morpholino-4-carbonyl; ($C_3$-$C_9$)-Cycloalkylcarbonyl; ($C_6$-$C_{10}$)-Aryl-($C_2$-$C_{11}$)-alkanoyl; 2-Pyridyl-($C_1$-$C_8$)-alkanoyl; 3-Pyridyl-($C_1$-$C_8$)-alkanoyl; 4-Pyridyl-($C_1$-$C_8$)-alkanoyl; gegebenenfalls durch Halogen, ($C_1$-$C_7$)-Alkyl, ($C_1$-$C_7$)-Alkoxy oder ($C_1$-$C_7$)-Alkoxycarbonyl substituiertes Benzoyl; Pyrrolyl-2-carbonyl; Pyridyl-3-carbonyl; Benzolsulfonyl; ($C_1$-$C_{10}$)-Alkoxycarbonyl; substituiertes ($C_1$-$C_{10}$)-Alkoxycar-

bonyl oder ($C_6$-$C_{14}$)-Aryl-($C_1$-$C_6$)-alkoxycarbonyl bedeutet,
$R^2$ Wasserstoff oder Methyl bedeutet, oder gemeinsam mit $R^3$ und der diese Reste tragenden -N-CH-Gruppe ein Tetrahydroisochinolin oder Azabicyclooctan-Gerüst bildet,
$R^3$ und $R^8$ unabhängig voneinander Wasserstoff, Methyl, Ethyl, Isopropyl, n-Propyl, n-Butyl, Isobutyl, sec.-Butyl, 3-Guanidinopropyl, Carbamoylmethyl, 2-Carbamoylethyl, Carboxymethyl, 2-Carboxyethyl, Mercaptomethyl, 2-(Methylthio)ethyl, (1-Mercapto,1-methyl)ethyl, Hydroxymethyl, 1-Hydroxyethyl, Amino, Aminomethyl, 2-Aminoethyl, 3-Aminopropyl, 4-Aminobutyl, N,N-Dimethylamino, Cyclohexylmethyl, Imidazol-4-yl-methyl, Benzyl, 2-Methylbenzyl, 3-Methylbenzyl, Indol-3-yl-methyl, 4-Hydroxybenzyl, 4-Methoxybenzyl, 3,4-Dihydroxybenzyl, 3,4-Dimethoxybenzyl, (Benzdioxolan-5-yl)methyl, 2-Thienyl, 2-Thienylmethyl, 2-(2-Thienyl)ethyl, 3-Thienyl, 3-Thienylmethyl, 2-(3-Thienyl)ethyl, 4-Chlorbenzyl, 2-(Methylsulfinyl)ethyl, 2-(Methylsulfonyl)ethyl, 2-Pyridylmethyl, 3-Pyridylmethyl, 4-Pyridylmethyl, Cyclohexyl, (1-Methyl-imidazol-4-yl)methyl, (3-Methyl-imidazol-4-yl)methyl, Phenyl, 1-Naphthylmethyl, 2-Naphthylmethyl, 2-Phenylethyl, 2-Thiazolylmethyl, 4-Thiazolylmethyl, 3-Pyrazolylmethyl, 4-Pyrimidinylmethyl, Indol-2-yl-methyl, 2-Benzo[b]thienylmethyl, 3-Benzo[b]thienylmethyl, 2-Furylmethyl sind
oder mit $R^2$ bzw. $R^{19}$ Ringsysteme wie vorstehend unter $R^2$ definiert, bilden,
$R^4$ ($C_3$-$C_{12}$)-Alkyl; mono- oder bicyclisches ($C_3$-$C_{12}$)-Cycloalkyl oder ($C_3$-$C_{12}$)-Cycloalkylmethyl, wobei der Cycloalkylteil gegebenenfalls durch ($C_1$-$C_4$)-Alkyl substituiert ist; ($C_6$-$C_{10}$)-Arylmethyl; Dithiolanyl; Dithiolanylmethyl; Dithianyl und Dithianylmethyl ist,
$R^5$ Wasserstoff, Azido, Azidomethyl, 2-Azidoethyl, ($C_1$-$C_6$)-Alkyl, ($C_3$-$C_{12}$)-Cycloalkyl, ($C_3$-$C_{12}$)-Cycloalkyl-($C_1$-$C_3$)-alkyl, 3-(2-Pyridyl)-propyl, 3-(3-Pyridyl)-propyl, Imidazol-2-yl, Imidazol-1-yl, Imidazol-4-yl, 3-(4-Pyridyl)-propyl, (Imidazol-2-yl)-propyl, (Imidazol-1-yl)-propyl, (Imidazol-4-yl)-propyl, [1-($C_1$-$C_4$)-Alkylimidazol-2-yl]-propyl, (Imidazolin-2-yl)-propyl ist,
$R^6$ und $R^7$ wie in Anspruch 2 definiert sind,
$R^9$ Wasserstoff oder Methyl bedeutet, oder zusammen mit $R^8$ und der diese Reste tragenden -N-CH-Gruppe ein Tetrahydroisochinolin- oder Azabicyclooctan-Gerüst bildet,
$R^{10}$ Wasserstoff bedeutet oder zusammen mit $R^1$ und den diese tragenden Atomen ein mono- oder bicyclisches gesättigtes oder teilweise ungesättigtes Ringsystem mit 5-12 Ringgliedern bildet, das außer Kohlenstoff noch 1 Schwefelatom enthält, welches besonders bevorzugt zum Sulfon oxydiert ist, oder zusammen mit $R^8$ und den diese tragenden Atomen ein Thiochromansystem bildet, dessen Schwefelatom besonders bevorzugt zum Sulfon oxydiert ist,
$R^{11}$ und $R^{12}$ unabhängig voneinander Wasserstoff, Hydroxy, Phenyl, 2-Thienyl, 2-, 3- oder 4-Pyridyl, 1- oder 2-Imidazolyl, 1-Naphthyl, 2- oder 3-Benzo[b]thienyl sind,
n und m unabhängig voneinander 0, 1 oder 2 sind,
$R^{13}$ Wasserstoff oder ($C_1$-$C_4$)-Alkyl ist,
$R^{14}$ und $R^{15}$ wie in Anspruch 2 definiert sind, sowie deren physiologisch verträgliche Salze.

4. Verfahren zur Herstellung einer Verbindung der Formel I gemäß einem oder mehreren der Ansprüche 1 - 3, dadurch gekennzeichnet, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

5. Verbindung der Formel I gemäß einem oder mehreren der Ansprüche 1 - 3 zur Anwendung als Heilmittel.

6. Verbindung der Formel I gemäß einem oder mehreren der Ansprüche 1 - 3 zur Anwendung als Heilmittel bei dar Behandlung des Bluthochdrucks.

7. Pharmazeutisches Mittel enthaltend eine Verbindung der Formel I gemäß einem oder mehreren der Ansprüche 1 - 3.

Patentansprüche für folgende Vertragsstaaten: ES, GR

1. Verfahren zur Herstellung einer Verbindung der Formel I

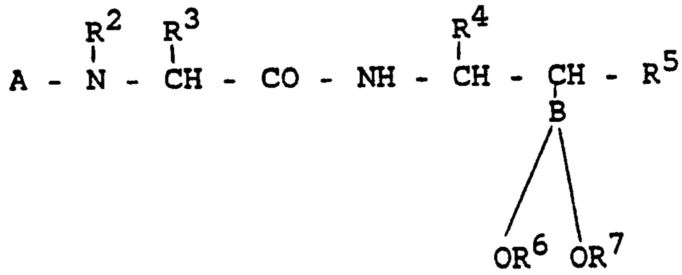

(I)

in welcher
A einen Rest der Formeln II, III oder IV bedeutet

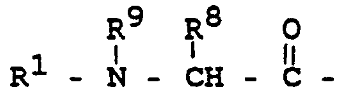

(II)

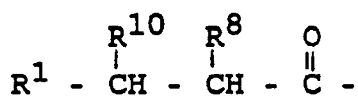

(III)

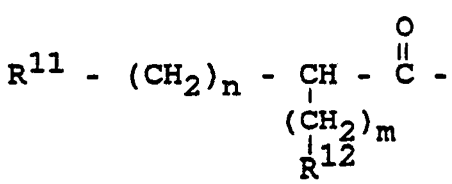

(IV)

worin

$R^1$ $a_1$) Wasserstoff, ($C_1$-$C_{12}$)-Alkyl, das gegebenenfalls ein-oder zweifach ungesättigt ist und das gegebenenfalls durch bis zu 3 gleiche oder verschiedene Reste aus der Reihe Hydroxy, ($C_1$-$C_7$)-Alkoxy, Carbamoyl, ($C_1$-$C_8$)-Alkanoyloxy, Carboxy, ($C_1$-$C_7$)-Alkoxycarbonyl, F, Cl, Br, I, Amino, Amidino, das gegebenenfalls durch einen, zwei oder drei ($C_1$-$C_8$)-Alkylreste substituiert sein kann, Guanidino, das gegebenenfalls durch einen, zwei, drei oder vier ($C_1$-$C_8$)-Alkylreste substituiert sein kann, ($C_1$-$C_7$)-Alkylamino, Di-($C_1$-$C_7$)-alkylamino, ($C_1$-$C_5$)-Alkoxycarbonylamino, ($C_7$-$C_{15}$)-Aralkoxycarbonylamino und 9-Fluorenylmethoxycarbonylamino substituiert ist; mono-, bi- oder tricyclisches ($C_3$-$C_{18}$)-Cycloalkyl, ($C_3$-$C_{18}$)-Cycloalkyl-($C_1$-$C_6$)-alkyl oder ($C_6$-$C_{14}$)-Aryl, wobei der Cycloalkylteil gegebenenfalls durch ($C_1$-$C_6$)-Alkyl substituiert ist und Aryl gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino und Trifluormethyl substituiert ist; ($C_6$-$C_{14}$)-Aryl-($C_1$-$C_6$)-alkyl, worin der Arylteil gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino, ($C_1$-$C_7$)-Alkylamino, Di-($C_1$-$C_7$)-alkylamino, Carboxy, Carboxymethoxy, Amino-($C_1$-$C_7$)-alkyl, ($C_1$-$C_7$)-Alkylamino-($C_1$-$C_7$)-alkyl, Di-($C_1$-$C_7$)-alkylamino-($C_1$-$C_7$)-alkyl, ($C_1$-$C_7$)-Alkoxycarbonylmethoxy, Carbamoyl, Sulfamoyl, ($C_1$-$C_7$)-Alkoxysulfonyl, Sulfo- und Guanidino-($C_1$-$C_8$)-alkyl substituiert ist; oder für den Rest eines 5- oder 6-gliedrigen monocyclischen oder 9-oder 10-gliedrigen bicyclischen teilweise oder vollständig hydrierten Heteroaromaten, mit mindestens 1 C-Atom, 1 - 4 N-Atomen und/oder 1 - 2 S-Atomen und/oder 1 - 2 O-Atomen als Ringglieder steht, und der gegebenenfalls wie ($C_6$-$C_{14}$)-Aryl unter $a_1$) definiert, mono-, di- oder trisubstituiert ist, bedeutet

oder

$a_2$) einen Rest der Formel V bedeutet

$R^1$ - W , (V)

worin $R^1$ wie $R^1$ unter $a_1$) definiert ist und W für

-CO-, -O-CO-, $-SO_2-$, -SO-, $-NH-SO_2-$, -NH-CO-, -CH(OH)-oder -N(OH)- steht;

$R^2$ Wasserstoff oder ($C_1$-$C_8$)-Alkyl bedeutet, oder zusammen mit $R^3$ und den diese tragenden Atomen ein mono- oder bicyclisches, gesättigtes oder teilweise ungesättigtes Ringsystem mit 5-12 Ringgliedern bildet; $R^3$ und $R^8$ unabhängig voneinander wie $R^1$ und $a_1$) definiert sind oder zusammen mit $R^2$ bzw. mit $R^9$ und den diese tragenden Atomen Ringsysteme mit 5-12 Ringgliedern bilden wie oben definiert;
$R^4$ ($C_3$-$C_{12}$)-Alkyl, mono-, bi- oder tricyclisches ($C_3$-$C_{18}$)-Cycloalkyl, ($C_3$-$C_{18}$)-Cycloalkylmethyl, ($C_3$-$C_{18}$)-Cycloalkylethyl, wobei der Cycloalkylteil gegebenenfalls durch ($C_1$-$C_6$)-Alkyl substituiert ist; Dithiolanyl; ($C_6$-$C_{14}$)-Arylmethyl; Dithiolanylmethyl; Dithiolanylethyl; Dithianyl; Dithianylmethyl oder Dithianylethyl bedeutet;
$R^5$ Wasserstoff; Azido; Azido-($C_1$-$C_4$)-alkyl; ($C_1$-$C_{12}$)-Alkyl, das gegebenenfalls durch Hydroxy, Azido oder Hydroxy und Azido substituiert sein kann; ($C_3$-$C_{12}$)-Cycloalkyl; ($C_3$-$C_{12}$)-Cycloalkyl-($C_1$-$C_6$)-alkyl; $C_3$-$C_{12}$)-Cycloalkylsulfonyl- ($C_1$-$C_6$)-alkyl; ($C_1$-$C_6$)-Alkylsulfonyl-($C_1$-$C_6$)-alkyl; ($C_6$-$C_{14}$)-Aryl, ($C_6$-$C_{14}$)-Aryl-($C_1$-$C_6$)-alkyl, wobei Aryl auch für den Rest eines 5- oder 6-gliedrigen monocyclischen oder 9-oder 10-gliedrigen bicyclischen Heteraromaten mit mindestens 1 C-Atom, 1-4 N-Atomen und/oder 1-2 S-Atomen und/oder 1-2 O-Atomen als Ringglieder stehen kann, der auch teilweise oder vollständig hydriert sein kann, ebenfalls ist es möglich, daß Aryl und Heteroaryl wie unter $a_1$) definiert substituiert sein kann,
$R^6$ und $R^7$ unabhängig voneinander Wasserstoff oder ($C_1$-$C_6$)-Alkyl bedeuten; oder zusammen mit dem Boratom und den Sauerstoffatomen ein mono-, bi- oder tricyclisches, gesättigtes oder teilweise ungesättigtes, mono-, di-, tri- oder tetra-($C_1$-$C_6$)-alkyliertes oder -phenyliertes Ringsystem mit 5-18 Ringgliedern, das außer dem Boratom, den Sauerstoffatomen und dem/den Kohlenstoffatom(en) noch ein -O- Glied, ein -$NR^{13}$-Glied oder ein -$CR^{14}R^{15}$-Glied enthalten kann, bilden;
$R^9$ für Wasserstoff oder ($C_1$-$C_8$)-Alkyl steht, oder zusammen mit $R^8$ und den diese tragenden Atomen ein mono- oder bicyclisches, gesättigtes oder teilweise ungesättigtes Ringsystem mit 5-12 Ringgliedern bildet;
$R^{10}$ Wasserstoff oder ($C_1$-$C_8$)-Alkyl ist, oder zusammen mit $R^1$ oder $R^8$ und den diese tragenden Atomen ein mono- oder bicyclisches, gesättigtes oder teilweise ungesättigtes Ringsystem mit 5-12 Ringgliedern bildet, das außer Kohlenstoff noch 1 Schwefelatom enthalten kann, welches gegebenenfalls zum Sulfoxid oder Sulfon oxidiert sein kann;
$R^{11}$ und $R^{12}$ unabhängig voneinander Wasserstoff, Hydroxy oder ($C_6$-$C_{14}$)-Aryl bedeuten, wobei Aryl gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino, ($C_1$-$C_7$)-Alkylamino, Di-($C_1$-$C_7$)-alkylamino, Carboxy, Carboxymethoxy, Amino- ($C_1$- $C_7$)-alkyl, ($C_1$-$C_7$)-Alkylamino-($C_1$-$C_7$)-alkyl, Di-($C_1$-$C_7$)-alkylamino-($C_1$-$C_7$)-alkyl, ($C_1$-$C_7$)-Alkoxycarbonylmethoxy, Carbamoyl, Sulfamoyl, ($C_1$-$C_7$)-Alkoxysulfonyl, Sulfo- und Guanidino-($C_1$-$C_8$)-alkyl substituiert ist; oder für den Rest eines 5- oder 6-gliedrigen monocyclischen oder 9- oder 10-gliedrigen bicyclischen, gegebenenfalls teilweise oder vollständig hydrierten Heteroaromaten, mit mindestens 1 C-Atom, 1 - 4 N-Atomen und/oder 1 - 2 S-Atomen und/oder 1 - 2 O-Atomen als Ringglieder stehen, der gegebenenfalls wie oben ($C_6$-$C_{14}$)-Aryl mono- oder disubstituiert ist,
n und m unabhängig voneinander 0, 1, 2, 3 und 4 sein können,
$R^{13}$ Wasserstoff oder ($C_1$-$C_{12}$)-Alkyl, das gegebenenfalls ein- bis zweifach ungesättigt ist und das gegebenenfalls durch bis zu 3 gleiche oder verschiedene Reste aus der Reihe Hydroxy, ($C_1$-$C_7$)-Alkoxy, Amino, Mono- oder Di-($C_1$-$C_7$)-alkylamino substituiert ist, bedeutet,
$R^{14}$ und $R^{15}$ unabhängig voneinander Wasserstoff, ($C_1$-$C_8$)-Alkyl, Hydroxymethyl, 2-Hydroxyethyl, (3-Hydroxysulfonyl, 2-hydroxypropyl)amino, (2-Hydroxysulfonylethyl) amino, (2-Hydroxysulfonylpropyl)amino, (Carboxymethyl)amino, Bis(2-hydroxyethyl)amino bedeuten
sowie deren physiologisch verträgliche Salze, dadurch gekennzeichnet, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

2. Verfahren gemäß Anspruch 1, dadurch gekennzeichnet, daß
$R^1$ Wasserstoff bedeutet oder für ($C_1$-$C_{10}$)-Alkyl; Cyclopentyl; Cyclohexyl; Cyclopentyl-($C_1$-$C_{10}$)-alkyl; Cyclohexyl-($C_1$-$C_{10}$)-alkyl; gegebenenfalls substituiertes Phenyl-($C_1$-$C_8$)-alkyl; 2-Pyridyl-($C_1$-$C_8$)-alkyl; 3-Pyridyl-($C_1$-$C_8$)-alkyl; 4-Pyridyl-($C_1$-$C_8$)-alkyl; $H_2N$-($C_1$-$C_{10}$)-Alkyl; HO-($C_1$-$C_{10}$)-Alkyl; ($C_1$-$C_4$)-Alkoxy-($C_1$-$C_{10}$)-alkyl; ($C_1$-$C_4$)-Alkoxycarbonyl-($C_1$-$C_{10}$)-alkyl; ($C_1$-$C_8$)-Alkylsulfonyl; ($C_1$-$C_8$)-Alkylsulfinyl; ($C_1$-$C_8$)-Hydroxyalkylsulfonyl; ($C_1$-$C_8$)-Hydroxyalkylsulfinyl; Hydroxy-($C_1$-$C_{10}$)-alkanoyl; ($C_1$-$C_8$)-Alkanoyloxy-($C_1$-$C_{10}$)-alkyl; ($C_1$-$C_{11}$)-Alkanoyl; gegebenenfalls geschütztes Amino-($C_1$-$C_{11}$)-alkanoyl; Di-($C_1$-$C_7$)-alkylamino-($C_2$-$C_{11}$)-alkanoyl; Piperidino-4-carbonyl; Morpholino-4-carbonyl; ($C_3$-$C_9$)-Cycloalkylcarbonyl; ($C_6$-$C_{10}$)-Aryl-($C_2$-$C_{11}$)-alkanoyl; 2-Pyridyl-($C_1$-$C_8$)-alkanoyl; 3-Pyridyl-($C_1$-$C_8$)-alkanoyl; 4-Pyridyl-($C_1$-$C_8$)-alkanoyl; gegebenenfalls durch Halogen, ($C_1$-$C_7$)-Alkyl, ($C_1$-$C_7$)-Alkoxy oder ($C_1$-$C_7$)-Alkoxycarbonyl substituiertes Benzoyl; Pyrrolyl-2-carbonyl, Pyridyl-3-carbonyl; Benzolsulfonyl; ($C_1$-$C_{10}$)-Alkoxycarbonyl; substituiertes ($C_1$-$C_{10}$)-

Alkoxycarbonyl; ($C_6$-$C_{14}$)-Aryl-($C_1$-$C_6$)-alkoxycarbonyl steht, oder gemeinsam mit $R^{10}$ ein mono-oder bicyclisches, gesättigtes oder teilweise ungesättigtes Ringsystem mit 5-12 Ringgliedern, das außer Kohlenstoff noch 1 Schwefelatom enthalten kann, welches gegebenenfalls zum Sulfoxid oder Sulfon oxydiert sein kann,

$R^2$ Wasserstoff, Methyl oder Ethyl ist oder zusammen mit $R^3$ und den diese tragenden Atomen Pyrrolidin oder Piperidin bildet, die jeweils noch mit Cyclopentyl, Cyclohexyl oder Phenyl anneliert sein können,

$R^3$ und $R^8$ unabhängig voneinander Wasserstoff;

($C_1$-$C_{10}$)-Alkyl, daß gegebenenfalls ein- oder zweifach ungesättigt ist und das gegebenenfalls durch bis zu 3 gleiche oder verschiedene Reste aus der Reihe Hydroxy, ($C_1$-$C_7$)-Alkoxy, ($C_1$-$C_7$)-Alkanoyloxy, Carboxy, ($C_1$-$C_7$)-Alkoxycarbonyl, Cl, Br, Amino, Amidino, Guanidino, Carbamoyl, ($C_1$-$C_5$)-Alkoxycarbonylamino, ($C_6$-$C_{15}$)-Aralkoxycarbonylamino und 9-Fluorenylmethoxycarbonylamino substituiert ist; ($C_3$-$C_{12}$)-Cycloalkyl, ( $C_3$-$C_{12}$)-Cycloalkyl-($C_1$-$C_3$)-alkyl, mono- oder bicyclisches ($C_6$-$C_{14}$)-Aryl-($C_1$-$C_3$)-Alkyl, das gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino und Trifluormethyl substituiert ist, bedeutet oder für ($C_1$-$C_3$)-Alkyl, substituiert mit dem Rest eines 5- oder 6-gliedrigen monocyclischen oder 9- oder 10-gliedrigen bicyclischen, gegebenenfalls teilweise oder vollständig hydrierten Heteroatomen, mit mindestens 1 C-Atom, 1 - 4 N-Atomen und/oder 1 - 2 S-Atomen und/oder 1 - 2 O-Atomen als Ringglieder, der gegebenenfalls wie in Anspruch 1 für den Arylteil beschrieben mono- oder disubstituiert ist stehen, oder zusammen mit $R^2$ bzw. $R^9$ Ringsysteme, wie vorstehend unter $R^2$ beschrieben, bilden;

$R^4$ ($C_3$-$C_{12}$)-Alkyl; mono- bi- oder tricyclisches ($C_3$-$C_{18}$)-Cycloalkyl oder ($C_3$-$C_{18}$)-Cycloalkylmethyl, wobei der Cycloalkylteil gegebenenfalls durch ($C_1$-$C_4$)-Alkyl substituiert ist; ($C_6$-$C_{14}$)-Arylmethyl; Dithiolanyl; Dithiolanylmethyl; Dithianyl und Dithianylmethyl ist;

$R^5$ für Wasserstoff, Azido, Azido-($C_1$-$C_4$)-alkyl, ($C_1$-$C_{12}$)-Alkyl, ($C_3$-$C_{12}$)-Cycloalkyl, ($C_3$-$C_{12}$)-Cycloalkyl-($C_1$-$C_6$)-alkyl, (2-Pyridyl)-($C_1$-$C_4$)-alkyl, (3-Pyridyl)-($C_1$-$C_4$)-alkyl, (4-Pyridyl)-($C_1$-$C_4$)-alkyl, Imidazol-2-yl, Imidazol-1-yl, Imidazol-4-yl, (Imidazol-2-yl)-($C_1$-$C_4$)-alkyl, (Imidazol-1-yl)-($C_1$-$C_4$)-alkyl, (Imidazol-4-yl)-($C_1$-$C_4$)-alkyl, [1-($C_1$-$C_6$)-Alkylimidazol-2-yl]-($C_1$-$C_4$)-alkyl, (Imidazolin-2-yl)-($C_1$-$C_4$)-alkyl, [1-($C_1$-$C_6$)-Alkylimidazolin-2-yl]-($C_1$-$C_4$)-alkyl steht;

$R^6$ und $R^7$ unabhängig voneinander Wasserstoff sind oder zusammen mit dem Boratom und den Sauerstoffatomen ein mono-, bi- oder tricyclisches, gesättigtes oder teilweise ungesättigtes mono-, di-, tri- oder tetraalkyliertes oder -phenyliertes Ringsystem mit 5-18 Ringgliedern bilden, das außer dem Boratom, den Sauerstoffatomen und dem (den) Kohlenstoffatom(en) noch ein -O-Glied, ein -$NR^{13}$-Glied oder ein -$CR^{14}R^{15}$-Glied enthalten kann;

$R^9$ Wasserstoff, Methyl oder Ethyl ist oder zusammen mit $R^8$ und den diese tragenden Atomen Pyrrolidin oder Piperidin bilden, die jeweils zusätzlich mit Cyclopentyl, Cyclohexyl oder Phenyl anneliert sein können;

$R^{10}$ Wasserstoff oder Methyl ist, oder gemeinsam mit $R^1$ oder $R^8$ und den diese tragenden Atomen ein mono- oder bicyclisches gesättigtes oder teilweise ungesättigtes Ringsystem mit 5-12 Ringgliedern bilden, das außer Kohlenstoff noch 1 Schwefelatom enthalten kann, welches gegebenenfalls zum Sulfoxid oder Sulfon oxidiert sein kann,

$R^{11}$ und $R^{12}$ unabhängig voneinander Wasserstoff, Hydroxy, Phenyl, 2- oder 3-Thienyl, 2-, 3- oder 4-Pyridyl, 1-, 2-oder 4-Imidazolyl, 1- oder 2-Naphthyl; 2- oder 3-Benzo[b]thienyl sind;

n und m unabhängig voneinander 0, 1, 2, 3 und 4 bedeuten;

$R^{13}$ Wasserstoff oder ($C_1$-$C_{12}$)-Alkyl ist,

$R^{14}$ und $R^{15}$ unabhängig voneinander Wasserstoff, Hydroxymethyl, 2-Hydroxyethyl, (3-Hydroxysulfonyl, 2-hydroxypropyl)amino, (2-Hydroxysulfonylethyl)amino, (2-Hydroxysulfonylpropyl)amino, (Carboxymethyl)-amino, Bis(2-hydroxyethyl)amino bedeuten.

3. Verfahren gemäß Anspruch 1 oder 2, dadurch gekennzeichnet, daß

$R^1$ ($C_1$-$C_8$)-Alkylsulfonyl; ($C_1$-$C_8$)-Alkylsulfinyl; ($C_1$-$C_8$)-Hydroxyalkylsulfonyl; Hydroxy-($C_1$-$C_{10}$)-alkanoyl; ($C_1$-$C_8$)-Alkanoyloxy-($C_1$-$C_{10}$)-alkyl; ($C_1$-$C_{11}$)Alkanoyl; Amino-($C_1$-$C_{11}$)-alkanoyl; Di-($C_1$-$C_7$)-alkylamino-($C_2$-$C_{11}$)-alkanoyl; Piperidino-4-carbonyl; Morpholino-4-carbonyl; ($C_3$-$C_9$)-Cycloalkylcarbonyl; ($C_6$-$C_{10}$)-Aryl-($C_2$-$C_{11}$)-alkanoyl; 2-Pyridyl-($C_1$-$C_8$)-alkanoyl; 3-Pyridyl-($C_1$-$C_8$)-alkanoyl; 4-Pyridyl-($C_1$-$C_8$)-alkanoyl; gegebenenfalls durch Halogen, ($C_1$-$C_7$)-Alkyl, ($C_1$-$C_7$)-Alkoxy oder ($C_1$-$C_7$)-Alkoxycarbonyl substituiertes Benzoyl; Pyrrolyl-2-carbonyl; Pyridyl-3-carbonyl; Benzolsulfonyl; ($C_1$-$C_{10}$)-Alkoxycarbonyl; substituiertes ($C_1$-$C_{10}$)-Alkoxycarbonyl oder ($C_6$-$C_{14}$)-Aryl-($C_1$-$C_6$)-alkoxycarbonyl bedeutet,

$R^2$ Wasserstoff oder Methyl bedeutet, oder gemeinsam mit $R^3$ und der diese Reste tragenden -H-CH-Gruppe ein Tetrahydroisochinolin oder Azabicyclooctan-Gerüst bildet,

$R^3$ und $R^8$ unabhängig voneinander Wasserstoff, Methyl, Ethyl, Isopropyl, n-Propyl, n-Butyl, Isobutyl, sec.-Butyl, 3-Guanidinopropyl, Carbamoylmethyl, 2-Carbamoylethyl, Carboxymethyl, 2-Carboxyethyl, Mercaptomethyl, 2-(Methylthio)ethyl, (1-Mercapto,1-methyl)ethyl, Hydroxymethyl, 1-Hydroxyethyl, Amino, Aminomethyl, 2-Aminoethyl, 3-Aminopropyl, 4-Aminobutyl, N,N-Dimethylamino, Cyclohexylmethyl, Imidazol-4-yl-methyl, Benzyl, 2-Methylbenzyl, 3-Methylbenzyl, Indol-3-yl-methyl, 4-Hydroxybenzyl, 4-Methoxybenzyl, 3,4-Dihydroxybenzyl, 3,4-Dimethoxybenzyl, (Benzdioxolan-5-yl)methyl, 2-Thienyl, 2-Thienylmethyl, 2-(2-Thienyl)ethyl, 3-Thienyl, 3-Thienylmethyl, 2-(3-Thienyl)ethyl, 4-Chlorbenzyl, 2-(Methylsulfinyl)ethyl, 2-(Methylsulfonyl)ethyl, 2-Pyridylmethyl, 3-Pyridylmethyl, 4-Pyridylmethyl, Cyclohexyl, (1-Methyl-imidazol-4-yl)methyl, (3-Methyl-imidazol-4-yl)methyl, Phenyl, 1-Naphthylmethyl, 2-Naphthylmethyl, 2-Phenylethyl, 2-Thiazolylmethyl, 4-Thiazolylmethyl, 3-Pyrazolylmethyl, 4-Pyrimidinylmethyl, Indol-2-yl-methyl, 2-Benzo[b]-thienylmethyl, 3-Benzo[b]thienylmethyl, 2-Furylmethyl sind oder mit $R^2$ bzw. $R^{19}$ Ringsysteme wie vorstehend unter $R^2$ definiert, bilden,

$R^4$ $(C_3-C_{12})$-Alkyl; mono- oder bicyclisches $(C_3-C_{12})$-Cycloalkyl oder $(C_3-C_{12})$-Cycloalkylmethyl, wobei der Cycloalkylteil gegebenenfalls durch $(C_1-C_4)$-Alkyl substituiert ist; $(C_6-C_{10})$-Arylmethyl; Dithiolanyl; Dithiolanylmethyl; Dithianyl und Dithianylmethyl ist,

$R^5$ Wasserstoff, Azido, Azidomethyl, 2-Azidoethyl, $(C_1-C_6)$-Alkyl, $(C_3-C_{12})$-Cycloalkyl, $(C_3-C_{12})$-Cycloalkyl-$(C_1-C_3)$-alkyl, 3-(2-Pyridyl)-propyl, 3-(3-Pyridyl)-propyl, Imidazol-2-yl, Imidazol-1-yl, Imidazol-4-yl, 3- (4-Pyridyl)-propyl, (Imidazol-2-yl)-propyl, (Imidazol-1-yl)-propyl, (Imidazol-4-yl)-propyl, [1- $(C_1-C_4)$-Alkylimidazol-2-yl]-propyl, (Imidazolin-2-yl)-propyl ist,

$R^6$ und $R^7$ wie in Anspruch 2 definiert sind,

$R^9$ Wasserstoff oder Methyl bedeutet, oder zusammen mit $R^8$ und der diese Reste tragenden -N-CH-Gruppe ein Tetrahydroisochinolin- oder Azabicyclooctan-Gerüst bildet,

$R^{10}$ Wasserstoff bedeutet oder zusammen mit $R^1$ und den diese tragenden Atomen ein mono- oder bicyclisches gesättigtes oder teilweise ungesättigtes Ringsystem mit 5-12 Ringgliedern bildet, das außer Kohlenstoff noch 1 Schwefelatom enthält, welches besonders bevorzugt zum Sulfon oxydiert ist, oder zusammen mit $R^8$ und den diese tragenden Atomen ein Thiochromansystem bildet, dessen Schwefelatom besonders bevorzugt zum Sulfon oxydiert ist,

$R^{11}$ und $R^{12}$ unabhängig voneinander Wasserstoff, Hydroxy, Phenyl, 2-Thienyl, 2-, 3- oder 4-Pyridyl, 1- oder 2-Imidazolyl, 1-Naphthyl, 2- oder 3-Benzo[b]thienyl sind,

n und m unabhängig voneinander 0, 1 oder 2 sind,

$R^{13}$ Wasserstoff oder $(C_1-C_4)$-Alkyl ist,

$R^{14}$ und $R^{15}$ wie in Anspruch 2 definiert sind.

4. Verwendung einer nach einem der Verfahren der Ansprüche 1 bis 3 hergestellten Verbindung der Formel I als Heilmittel.

5. Verwendung einer nach einem der Verfahren der Ansprüche 1 bis 3 hergestellten Verbindung der Formel I zur Bluthochdrucktherapie und zur Behandlung der kongestiven Herzinsuffizienz.

6. Verfahren zur Herstellung einer pharmazeutischen Zubereitung enthaltend eine nach einem der Verfahren der Ansprüche 1 bis 3 hergestellten Verbindung der Formel I, dadurch gekennzeichnet, daß man diese zusammen mit einem physiologisch unbedenklichen Träger und gegebenenfalls weiteren Hilfs- und Zusatzstoffen in eine geeignete Darreichungsform bringt.